(19) Europäisches Patentamt
European Patent Office
Office européen des brevets

(11) Publication number: **0 156 643**
**B1**

(12) **EUROPEAN PATENT SPECIFICATION**

(45) Date of publication of patent specification: **08.08.90**

(21) Application number: **85302053.5**

(22) Date of filing: **25.03.85**

(51) Int. Cl.[5]: **C 07 J 5/00,** C 07 J 21/00, C 07 J 41/00, C 07 J 51/00, A 61 K 31/57 // C07J17/00

(54) **Ester prodrugs of steroids.**

(30) Priority: **28.03.84 US 594096**
**28.03.84 US 594097**
**14.02.85 US 701601**

(43) Date of publication of application:
**02.10.85 Bulletin 85/40**

(45) Publication of the grant of the patent:
**08.08.90 Bulletin 90/32**

(84) Designated Contracting States:
**BE CH DE FR GB IT LI NL SE**

(56) References cited:
**EP-A- 106 453**
**EP-A- 120 557**
**EP-A- 124 233**
**EP-A-0 010 056**
**FR-A-1 421 204**
**FR-A-1 601 117**
**GB-A-1 115 893**
**US-A-3 025 311**
**US-A-3 175 946**
**US-A-4 302 452**
**US-A-4 343 799**

(73) Proprietor: **THE UPJOHN COMPANY**
**301 Henrietta Street**
**Kalamazoo, Michigan 49001 (US)**

(72) Inventor: **Braughler, John Mark**
**c/o The Upjohn Compagny 301 Henrietta Street**
**Kalamazoo Michigan 49001 (US)**
Inventor: **Hall, Edward Dallas**
**c/o The Upjohn Compagny 301 Henrietta Street**
**Kalamazoo Michigan 49001 (US)**
Inventor: **McCall, John Michael**
**c/o The Upjohn Compagny 301 Henrietta Street**
**Kalamazoo Michigan 49001 (US)**
Inventor: **Wierenga, Wendell**
**c/o The Upjohn Compagny 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

**EP 0 156 643 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

Conventional corticosteroids, such as cortisone, hydrocortisone, prednisone, methylprednisolone, etc., are generally poorly water soluble and therefore not suited for intravenous administration. Several types of soluble C—21 derivatives of such steroids have been disclosed in the patent literature including dicarboxylic acid hemiesters, sulfobenzoates, sulfopropionates, sulfates, phosphates, and aminoalkanoyloxy derivatives. While solubilization can generally be attained quite readily using a variety of such pro-moities, most of the aforementioned derivatives possess other disadvantages limiting their utility as water soluble prodrugs. The term "prodrug" denotes a derivative of an active drug which is converted after administration back to the active drug. The "pro-moiety" referred to in this application is the fragment attached to the steroid via an ester linkage and removed by ester hydrolysis in vivo. A major problem with many common derivatives is their solution instability. Dicarboxylic acid hemiesters of corticosteroids such as succinate esters, for example, are marketed commercially as lyophilized powders for reconstitution prior to injection due to their solution instability (see, for example, E. R. Garrett, J. Pharm. Sci., *51*, 445 (1962) and J. Med. Pharm. Che. *5*, 112 (1962); B. D. Anderson and V. Taphouse, J. Pharm. Sci., *70*, (1981); R. Yamamoto, S. Fujisawa, and M. Kawamura, Yakugaku Zasshi, *91*, 855 (1971); B. D. Anderson, et. al., J. Pharm. Sci. *72*, 448 (1983)). Corticosteroid 21-aminoalkyl carboxylate derivatives reported in the literature also undergo rapid hydrolysis in aqueous solution (M. Kawamura, R. Yamamoto, and S. Fujisawa, Yakugaku Zasshi, *91*, 863 (1971)).

Certain derivatives which do appear to exhibit better solution stability suffer from other disadvantages. 21-sulfate esters, for example, may not be readily converted to the active parent drug in vivo as suggested by the fact that the 21-sulfate of hydrocortisone is inactive in mice (M. Kawamura, R. Yamamoto, and S. Fujisawa, Yakugaku Zasshi, *91*, 871 (1971); meta-sulfobenzoate esters which have been reported as having improved solution stability (M. Kawamura, R. Yamamoto and S. Fujisawa, ibid, French Patent Derwent No. 76199 U)) are frequently not highly water soluble and thus may have limited utility as injectable prodrugs. Phosphate esters may in some cases possess the requisite solubility, solution stability, and bioconversion rates but exhibit other disadvantages. Several undesirable features of phosphate esters are apparent: 1) Phosphate esters are often difficult to purify and are frequently very hygroscopic. 2) The stability of phosphate esters is optimum above pH 7 where other modes of drug degradation may be a problem. Glass surfaces are also more likely to delaminate in alkaline conditions resulting in particulate problems. 3) Precipitation of free corticosteroid due to the limited amount of hydrolysis which does occur may limit product shelf-life. Solubilization of free corticosteroid due to micelle formation by the intact prodrug is a desirable feature which phosphate esters exhibit to only a limited extent. 4) Concentrated solutions of phosphate esters of corticosteroids exhibit accelerated reaction velocities due to micelle formation, limiting shelf-life in concentrated solutions (G. L. Flynn and D. J. Lamb, J. Pharm. Sci. *59*, 1433 (1970)). Sulfopropionate esters of corticosteroids have also been reported as readily water soluble and as having improved solution stability (Derwent Accession No. 27789 C). Sulfoacetate esters are also known (Derwent 9453F). The present invention provides a novel class of ester prodrugs of corticosteroids which do not possess the typical glucocorticoid activity.

Field of Invention

The present invention is novel ester prodrugs of steroids and formulations thereof which have pharmacologically useful properties.

Summary of Invention

The compounds of the present invention are novel ester prodrugs of steroids which are solution stable in vitro but are rapidly converted in vivo to the active parent steroid and are therefore useful pharmacological agents. The compounds of the present invention are represented generically by general Formula I (see Formula Chart) wherein the various substituents have the following meanings:

wherein - - - indicates a single or double bond;

$R_1$ is $CH_3$ or $C_2H_5$;

either $R_2$ is H and $R_3$ is in the α-position and is OH, $C_{1-12}$ alkoxy, ($C_{1-4}$ alkyl)COO, aryl-COO, $(R)_2N$—COO or $R_7O$—COO, wherein the R's are independently selected from H and $C_{1-4}$ alkyl, $R_7$ is aryl or $C_{1-4}$ alkyl, and aryl is phenyl optionally substituted by 1 to 3 substituents selected from Cl, F, Br, $C_{1-3}$ alkyl, $C_{1-3}$ alkoxy, $C_{1-3}$ alkylthio, $Cl_3C$, $F_3C$, $NH_2$ and $NHCOCH_3$; or

$R_2$ is α-Cl and $R_3$ is β-Cl; or

$R_2$ and $R_3$ taken together are —O— bridging positions C—9 and C—11 or form a double bond between positions C—9 and C—11;

$R_4$ is H, $CH_3$, Cl or F;

$R_5$ is H, OH, F, Cl, Br or $CH_3$;

$R_6$ is H or $CH_3$;

$R_9$ is H, OH, $CH_3$, F or $=CH_2$;

either $R_{10}$ is H, OH or $CH_3$ or $R_{10}$ forms a second bond between positions C—16 and C—17; and

$R_{11}$ is $—Z(CH_2)_rQ$, $—Y—(CH_2)_n—X—(CH_2)_m—SO_3H$ or $—Y'—(CH_2)_p—X'—(CH_2)_q—NR_{12}R_{13}$, wherein Y is a

bond or —O—; Y' is a bond, —O— or —S— each of X and X' is a bond, —CON($R_{14}$)—, —N($R_{14}$)—, —N($R_{14}$)CO—, —O—, —S—, —S(O)—, or —S($O_2$)—; $R_{14}$ is hydrogen or $C_{1-4}$ alkyl; either each of $R_{12}$ and $R_{13}$ is ($C_1$—$C_4$) alkyl or ($C_1$—$C_4$) monohydroxyalkyl or $NR_{12}R_{13}$ is pyrrolidino, piperidino, morpholino, thiomorpholino, piperazino or N—($C_{1-4}$ alkyl)piperazino; n is an integer of from 2 to 9; m is an integer of from 1 to 5; p is an integer of from 2 to 9; q is an integer of from 1 to 5; Z is a bond or —O—; r is an integer of from 2 to 9; and Q is

(1) —$R_{15}$—$CH_2COOH$ wherein $R_{15}$ is —S—, —S(O)—, —S($O)_2$—, —$SO_2N(R_{16})$— or —N($R_{16}$)$SO_2$—, and $R_{16}$ is hydrogen or $C_{1-4}$ alkyl;

(2) —CO—COOH;

(3) —CONH—$CHR_{18}$—COOH wherein $R_{18}$ is H, $CH_3$, —$CH_2COOH$, —$CH_2CH_2COOH$, —$CH_2OH$, —$CH_2SH$, —$CH_2CH_2SCH_3$ or p-hydroxybenzyl;

(4) —$CONCH_3$—$CH_2$—COOH;

(5) (2-carboxy-1-pyrrolidinyl)carbonyl; or

(6) —CONH—$CH_2$—CONH—$CH_2COOH$;

with the provisos that:

(a) the total number of carbon atoms in $R_{16}$ and $(CH_2)_r$ is not greater than 10;

(b) when n is 2, $R_{14}$ is not H;

(c) the sum of m and n is not greater than 10:

(d) the sum of p and q is not greater than 10;

(e) when X is a bond, the sum of m and n is from 5 to 10;

(f) when X' is a bond, the sum of p and q is from 4 to 9;

(g) when $R_4$ is Cl or F, the C—1 position is saturated; and

(h) when $R_9$ is $=CH_2$, $R_{10}$ does not form a second bond between positions C—16 and C—17.

Included within the scope of the present invention are pharmacologically-acceptable salts of the compounds of Formula I. Solution stable formulations of the compounds of Formula I are also a part of the present invention.

Detailed Description of the Invention

Preferred embodiments of the present invention are set forth in formulae II to VI wherein the substituent groups have the followings meanings:

In Formulae II to VI each of $R_4$, $R_7$ and $R_8$ is hydrogen; $R_1$ is $CH_3$ or $C_2H_5$; $R_5$ is $CH_3$, F, Cl, Br, H or OH, and more preferably $R_5$ is in the α-position and is $CH_3$, H or F; $R_6$ is H or $CH_3$ and more preferably is H; $R_9$ is H, α-OH or $CH_3$; and $R_{10}$ is α-H or α-OH; and $R_{11}$ has the meaning defined in Formula I. Additionally in Formula III $R_2$ is hydrogen and $R_3$ is in the α-position and is OH, —O-akyl($C_1$—$C_{12}$), preferably —O-alkyl($C_1$—$C_4$), —O—COalkyl($C_1$—$C_{12}$), preferably —O—COalkyl($C_1$—$C_6$), —O—COaryl, —O—CON$(R)_2$ or —$OCOOR_7$ wherein aryl, R, and $R_7$ having the meanings defined in Formula I and preferably aryl is phenyl and R is hydrogen or methyl.

Pharmacologically acceptable salts of the compounds of Formula I include acid addition salts and quaternary ammonium salts when $R_{11}$ is Y'—$(CH_2)_p$—X'—$(CH_2)_q$—$NR_{12}R_{13}$. Illustrative examples of such acid addition salts are inorganic salts such as hydrochloride, hydrobromide, sulfate, or phosphate, or organic salts such as acetate, malonate, succinate or sulfonates. Quaternary ammonium salts of compounds of Formula I containing a terminal amine group may be depicted as follows where $R_{11}$ is

$$-Y'-(CH_2)_p-X'-(CH_2)_q-\underset{\underset{\text{alkyl}}{|}}{N}R_{12}R_{13}\quad R_8^{(-)}$$

wherein Y', p, X', q, $R_{12}$ and $R_{13}$ have the meanings defined in Formula I, alkyl has from 1 to 4 carbon atoms, and $R_8(^-)$ represents an anion, for example $R_8$ is I, Br, Cl, $CH_3SO_3$ or $CH_3COO$.

To form acid addition salts of the compounds of Formula I containing a terminal amine function, said compounds are treated with suitable pharmaceutically acceptable inorganic or organic acids by standard procedures. Suitable inorganic acids are, for example, hydrochloric, hydrobromic, nitric, sulfuric or phosphoric acids. Suitable organic acids include carboxylic acids, such as, acetic, propionic, glycolic, lactic, pyruvic, malonic, succinic, fumaric, malic, tartaric, citric, stearic, ascorbic, maleic, hydroxymaleic, benzoic, hydroxybenzoic, phenylacetic, glutamic, glutaric, cinnamic, salicylic, 2-phenoxybenzoic or sulfonic acids such as methane sulfonic, sulfonilic, toluenesulfonic, or 2-hydroxyethanesulfonic. The quaternary ammonium salts are formed by contacting said compounds with a suitable alkylating agent such as dimethyl sulfate, diethyl sulfate, or a suitable alkyl halide such as methyl or ethyl chloride or methyl or ethyl bromide or methyl or ethyl iodide.

Also included within the scope of this invention are base addition salts of compounds of Formule I wherein $R_{11}$ is —Y—$(CH_2)_n$—X—$(CH_2)_mSO_3H$ or —$Z(CH_2)_rQ$ which are obtained by treating the acid of Formula I with pharmaceutically acceptable inorganic or organic bases by standard procedures. Suitable inorganic bases are, e.g., those of alkali metal hydroxides, such as potassium, sodium, magnesium, and aluminum. Suitable organic bases are physiologically acceptable compounds containing tertiary amine

functional groups, e.g. choline($OH^-$), trishydroxymethylmethylamine, triethanolamine, or trialkylamines such as triethylamine.

The aryl moiety in the $R_3$ group —OCOaryl is attached to the carbonyloxy moiety through any of the available ring carbon atoms of said aryl moiety.

Any reference herein to compounds of Formula I includes pharmacologically acceptable salts thereof.

The compounds of the present invention are useful in treating the following diseases and injuries: head trauma, spinal trauma, septic or traumatic shock, stroke, and hemorrhagic shock. In addition utility in cancer as well as other disorders or physiological phenomenon dependent on aniogenesis such as embryo implantation (antifertility), arthritis, and atherosclerosis is exhibited with these compounds coadministered with oral heparin or systemic heparin fragments (see J. Folkman, et al., Science *32*, 719—725 (1983). The compounds of the present invention possess substantially none of the typical glucocorticoid effects.

The compounds of the present invention can be administered orally, intramuscularly, intravenously and by suppository, and the effective dosage range is 60 to 100 mg/kg/day. Additionally, a dosage regimen of using a loading dose of about 30 mg/kg followed by a repetitive as needed maintenance dose of about 15 mg/kg may be desirable. The compounds of the present invention may be coadministered with low doses of glucocorticoids. For the treatment of cancer including head tumors and other conditions dependent upon angiogenesis a preferred dosage range is 10 to 100 mg/kg/day and the preferred route of administration is orally, by suppository or intramuscularly.

The utility of the compounds of the present invention can be demonstrated in various test models as follows: For head trauma, mice are struck upon the head with a standard weight which is dropped from a set height. They are then dosed subcutaneously with the test compound. After one hour the motor abilities of the mice are assessed. Active test compounds promote improved motor activity relative to controls. For spinal trauma, see E. D. Hall and J. M. Braughler, Surg. Neurol. *18*, 320—327 (1982) and J. Neurosurg. *56*, 838—844 (1982). Septic (traumatic) shock is demonstrated in a rat model whereby test compound is administered and protection of the rats from the lethal effects of endotoxin is measured. For stroke, the carotid arteries of gerbils are ligated for a brief period after which test compound is administered subcutaneously. The behavior of the gerbils is observed after a recovery period, and gerbils receiving test compound display a more normal behavior after the recovery period. And for hemorrhagic shock, by published procedures used to evaluate glucocorticoids.

The novel compounds of the present invention provide marked advantages over known steroids in that these novel compounds are highly water soluble which facilitates formulation of the compounds and permits long term storage of solutions of said novel compounds.

The solution stability of these compounds is due to several features: 1) The derivatives are highly soluble in the pH range 3 to 6 which is the pH range in which ester hydrolysis in aqueous solution is minimized. 2) Functional groups which may promote ester hydrolysis through any catalytic or substituent effect are sufficiently distant from the ester linkage that such influences are minimized. 3) The compounds self-associate in concentrated solutions to form molecular aggregates which increase the shelf life of formulations by a) retarding hydroxide ion catalyzed ester hydrolysis at high concentrations, and b) solubilizing any parent steroid present in and resulting from the hydrolysis of a solution of a compound of the present invention.

For storage of aqueous solutions of the compounds of Formula I the pH of their solution must be properly controlled. Ideally, the pH will be maintained at a level where the hydrolysis of the ester is at a minimum. This minimum depends to a certain degree on the chemical structure of the pro-moiety, the formulation concentration, and the temperature of storage but in general will be at a pH of about 3 to 6 for the compounds of this invention. Most advantageously, buffers should be employed to maintain the pH at or near the desired level throughout the shelf life of the formulation. Suitable buffers are those which are physiologically acceptable and exhibit sufficient buffer capacity in the pH range 3—6, e.g., acetate, citrate, succinate, or phthalate buffers and the like. The quantity of buffer used is determined by means known in the art and will depend on the pH desired, the concentration of the solution, and the buffering capacity of the buffer.

The concentration of the solution stable formulations of the compounds of Formula I depends on the activity level of and the ultimate dose of parent steroid desired. In general the stability of the formulations increases as the concentration of novel ester increases. In essence the solution stable formulations may be as concentrated as viscosity properties permit or until the solubility of the novel ester is exceeded.

Sterile aqueous solutions of the compounds of Formula I typically will contain other components such as preservatives, anti-oxidants, chelating agents, or other stabilizers. Suitable preservatives can include benzyl alcohol, the parabens, benzalkonium chloride, or benzoic acid. Anti-oxidants such as sodium bisulfite, ascorbic acid, propyl 3,4,5-trihydroxy benzoate, and the like may be employed. Chelating agents such as citrate, tartrate, or ethylenediaminetetraacetic acid (EDTA) may be used. Other additives useful as stabilizers of corticosteroid prodrugs (e.g., creatinine, polysorbate 80, and the like) may be employed.

Sterile aqueous solutions of compounds of Formula I can be administrered to the patient being treated, i.e., a warm blooded mammal, intramuscularly or intravenously or orally. Additionally conventional solid dosage forms of the compounds of Formula I can be administered orally to the patient being treated. For example, capsules, pills, tablets or powders of the compounds of Formula I can be formulated in unit dosage forms incorporating conventional fillers, dispersants, preservatives and lubricants. Also

suppositories providing a sustained release of a compound of Formula I can be formulated using conventional inert materials such as biodegradable polymers or synthetic silicones.

The compounds of the present invention are prepared by various routes using conventional procedures. For convenience in describing the preparation of the compounds of Formula I the symbol St is employed to represent that portion of Formula I as depicted by Formula VII.

Preparation of Compounds of Formula I wherein $R_{11}$ is $—Y—(CH_2)_n—X—(CH_2)_mSO_3H$

When Y is oxy, i.e., —O—, equimolar amounts of an intermediate of the formula $O_2N(C_6H_4)—OCOO—(CH_2)_n—X—(CH_2)_mSO_3H$ (Formula VIII) wherein $(C_6H_4)$ is 1,4-phenylene and n, m, and X have the meanings defined in Formula I, and a parent steroid of the formula StOH wherein St has the meaning defined in Formula VII are reacted in a dry aprotic solvent such as tetrahydrofuran (THF), dimethylformamide (DMF) or dimethylsulfoxide (DMSO), in the presence of an acylation catalyst such as dimethylaminopyridine (DMAP) or N-methylimidazole. Although the reaction may be performed at room temperature it is convenient to gently warm the reaction mixture to about 50°—60°C with stirring until all the activated carbonate ester is consumed. The product is purified by pouring the reaction mixture into water with the pH adjusted to ~4 and washing with an organic solvent, e.g., ether or ethyl acetate. It is then concentrated by removing the solvent and further purified either as the free acid or as an appropriate salt by crystallization and/or chromatography.

When Y is a bond equimolar amounts of an intermediate of the formula

$$HOOC(CH_2)_n—X—(CH_2)_m—SO_3H \qquad \text{(Formula IX)}$$

wherein n, m, and X have the meanings defined in Formula I with a 21-iodo or 21-O-mesyl derivative of the parent steroid which may be represented respectively by the formulas St-Iodo (Formula X) and St-O-mesyl (Formula XI) wherein St has the meaning defined in Formula VII and mesyl means $—S(O_2)—CH_3$ are reacted. When the 21-iodo steroid derivative is employed the reaction proceeds at room temperature, whereas when the 21-O-mesyl steroid derivative is used the reaction is heated to about 60°—70°C. The reaction is carried out in a dry aprotic solvent such as DMF in the presence of a sterically hindered tertiary amine such as diisopropylethylamine. The product is isolated by diluting with water, adjusting the pH to ~5, washing with an organic solvent, suitably ethyl acetate, and further purifying by recrystallization or chromatography.

When Y is a bond and X is $—CON(R_{14})—$ compounds may also be prepared by reacting equimolar amounts of a 21-iodo steroid derivative of Formula XII and a bis-acid of the formula $HOOC—(CH_2)_n—COOH$ (Formula XII) wherein n has the meaning defined in Formula I in a dry aprotic solvent such as THF or DMF in the presence of a sterically hindered amine such as diisopropylethylamine with optional heating to give an intermediate of the formula $StOCO(CH_2)_n—COOH$ (Formula XIII) which is activated by cooling to about −20° to −10°C and reacting with isobutyl chloroformate in the presence of a tertiary amine, such as triethylamine for about 10—20 minutes during which time the reaction mixture is permitted to warm. To the activated derivative of Formula XIII is added an appropriate aminoalkylsulfonate of the formula

$$\overset{+}{R_{14}NH_2}—(CH_2)_mSO_3^- \qquad \text{Formula XIV}$$

wherein m and $R_{14}$ have the meanings defined in Formula I. This latter reaction is complete within an hour, and the product is isolated by standard procedures, e.g., washing an aqueous solution, pH 5, with an appropriate organic solvent such as ethyl acetate, and purification by crystallization and/or chromatography.

Alternatively when Y is a bond and X is $—CON(R_{14})—$ to the above obtained activated derivative of Formula XIII is added p-nitrophenol in the presence of a tertiary amine such as triethylamine to give a stable intermediate of the formula $StOCO(CH_2)_nCOO—(C_6H_4)—NO_2$ (Formula XV) wherein St and n have the meanings defined in Formula I and $(C_6H_4)$ is 1,4-phenylene. The intermediate of Formula XV is then reacted with a molar equivalent of an aminoalkylsulfonate of Formula XIV in a dipolar aprotic solvent such as THF or DMF in the presence of a base such as pyridine. The Formula I product is then isolated by washing an aqueous solution at pH 5 with an organic solvent, such as ethyl acetate, and purifying by crystallization and/or chromatography.

The compounds of Formula VIII wherein X is $—CON(R_{14})—$ are prepared by heating to about 60°C a suitable aliphatic lactone, such as, propiolactone, γ-butyrolactone, δ-valerolactone, ε-caprolactone, etc., as n in Formula I increases in length, with an equimolar amount of an ω-aminoalkylsulfonate of Formula XIV in an aprotic solvent such as DMSO, DMF or THF to give the acyclic amide which is isolated by standard extractive procedures. The amide is reacted with p-nitrophenylchloroformate in a dry aprotic solvent such as THF in the presence of pyridine and isolated by standard procedures to give the compounds of Formula VIII or used without isolation to form compounds of Formula I.

The compounds of Formula VIII wherein X is $—N(R_{14})—CO—$ are prepared by reacting an appropriate ω-sulfo alkanoic acid having an alkylene chain length of from 1 to 5 carbon atoms with an ω-amino alcohol of the formula $HO—(CH_2)_n—NHR_{14}$, wherein n and $R_{14}$ have the meanings defined in Formula I, in a dry aprotic solvent, such as THF or DMF, in the presence of dicyclohexylcarbodiimide (DCC) to yield the amide.

Any ester formed by reaction at the wrong end of the amino alcohol is eliminated by selective hydrolysis. Alternatively, a cyclic anhydride of Formula A (see Formula Chart) such as 3-sulfopropionic anhydride is reacted with an ω-amino alcohol in a polar aprotic solvent in the presence of a tertiary amine to form the amide. The product is isolated by standard extractive methods, and the product is taken up in a dry aprotic solvent and treated with p-nitrophenylchloroformate in the presence of pyridine to give the compounds of Formula VIII which may be isolated by standard procedures.

The compounds of Formula VIII wherein X is oxygen are prepared by reacting a suitable α,ω-aliphatic diol of the formula $HO(CH_2)_n$—OH wherein n has the meaning defined in Formula I with an ω-halosulfonate of formula Z'—$(CH_2)_mSO_3$— where Z' = Cl, Br, I, —O-mesyl, or —O-tosyl and m is as defined in Formula I, or, alternatively, with a sultone of Formula B (see Formula Chart) wherein m is as defined in Formula I, in a dry aprotic solvent in the presence of one equivalent of potassium t-butoxide to yield the desired ether. This compound is purified by standard extractive methods, then is reacted with p-nitrophenyl chloroformate in a dry aprotic solvent in the presence of pyridine to give a reactive mixed p-nitrophenyl carbonate ester of Formula VIII.

To prepare the compounds of Formula VIII wherein X is sulfur, an aliphatic ω-halo alcohol of the formula $HO(CH_2)_n$-halo wherein n is as defined in Formula I and halo is chloro, bromo, or iodo is reacted with thiourea in refluxing lower alcohol to yield an isothiouronium salt which is then cleaved by treating the compound with an aqueous base to yield an ω-mercaptoalkanol ($HS(CH_2)_nOH$—. The ω-mercapto alkanol, after isolation via standard methods, e.g., distillation, is then reacted with an ω-bromoalkylsulfonic acid of formula ($Br(CH_2)_mSO_3H$ wherein m is as defined in Formula I or a sultone of Formula B in a solution containing two equivalents of inorganic base in water. A water miscible solvent (e.g., alcohol) may also be added to solubilize the reactants. The product of formula $HO(CH_2)_nS(CH_2)_mSO_3$— is isolated by standard extractive procedures. Final purification is achieved by recrystallization and/or chromatography. This product may be oxidized at this stage to give a sulfoxide or sulfone if desired, or it may be maintained in the sulfide form. To form the sulfoxide, i.e., X is —S(O)—, the sulfide is treated with one equivalent of sodium metaperiodate in aqueous lower alcohol at 0°C. When oxidation is complete the sodium iodate is filtered out and the sulfoxide isolated by standard procedures. To form the sulfone, i.e., X is —$S(O_2)$—, the sulfide is reacted with 30% $H_2O_2$ in 50% acetic acid at room temperature for several hours. Oxidation proceeds through the sulfoxide to the sulfone. The product is isolated by standard procedures, with final purification being achieved by recrystallization or by chromatography if needed. The sulfur-linked hydroxyl containing sulfonate is then converted to a reactive mixed carbonate ester by combining it with an equimolar quantity of p-nitrophenylchloroformate in an aprotic solvent with added pyridine to give the compounds of Formula VIII which may be isolated by standard procedures.

The compounds of Formula VIII wherein X is a bond are prepared by reacting a sulfoalkanol of the formula $(HO(CH_2)_{n'}SO_3H$ (Formula XVI) wherein n is from 5 to 10 with p-nitrophenylchloroformate in a dry polar aprotic solvent such as DMF or DMSO in the presence of a tertiary amine such as triethylamine. The reaction product is isolated by standard procedures to give a compound of Formula VIII or is used without isolation to prepare compounds of Formula I.

The compounds of Formula XVI may be prepared by reacting an alcohol of the formula HO—$(CH_2)_{n'}$—$R_b$ wherein n' has the meaning defined in Formula XVI and $R_b$ is Cl, Br, I, $OS(O_2)CH_3$ or $OS(O_2)$—$(C_6H_4)$—$CH_3$ with a sulfite salt such as sodium sulfite in a mixture of water and a water miscible alcohol such as ethanol or propanol. The reaction mixture is heated to reflux and when the desired product formation has taken place, the product may be isolated by standard extractive methods and/or by crystallization.

Alternatively the compounds of Formula XVI may be synthesized in two steps involving the free radical addition of thiolacetic acid to a compound of the formula HO—$(CH_2)_{n'-2}$—$CH{=}CH_2$ wherein n' has the meaning defined in Formula I, followed by oxidation of the resulting thiolacetate with hydrogen peroxide in acetic acid to form compounds of Formula XVI. The addition reaction is carried out in the presence of ultraviolet radiation or a peroxide catalyst such as dibenzoyl peroxide. The oxidation is carried out in acetic acid to which 90% hydrogen peroxide has been added and is heated to 65 to 70°C. The products are isolated by standard methods.

The compounds of Formula IX wherein X is a bond are prepared by reacting a bromoalkanoate of the formula Br—$(CH_2)_{n'}$—$COO^-$ wherein n' is from 5 to 10 with a molar excess of a sulfite salt in refluxing water or a mixture of water and a water miscible alcohol. The product may be isolated by crystallization or by standard extractive methods. Alternatively the compounds of Formula IX wherein X is a bond may be obtained in two steps by first reacting a terminal olefin of the formula $CH_2{=}CH$—$(CH_2)_{n'-2}$—COOH wherein n' is from 5 to 10 with thiolacetic acid in the presence of ultraviolet radiation or a peroxide catalyst such as dibenzoyl peroxide under an inert atmosphere (e.g., $N_2$) to form a terminal thiolacetate of the formula $CH_2$—CO—S—$(CH_2)_{n'}$—COOH wherein n' is 5 to 10. The thiolacetate is isolated by standard methods and is then oxidized by treatment with hydrogen peroxide in acetic acid. The product of oxidation is a sulfoalkanoic acid of Formula IX which may be isolated by standard methods.

The compounds of Formula IX wherein X is —$N(R_{14})CO$— are prepared by reacting an amino acid of the formula $(HN(R_{14})(CH_2)_n$—COOH with a bromoalkanoyl chloride wherein the alkanoyl moiety contains from 2 to 6 carbon atoms in an aqueous solvent at a pH of about 10 after which the pH is adjusted to about 3. The thus formed amide is extracted with an organic solvent such as ethyl acetate and isolated by

procedures generally known in the art then taken up in aqueous alcohol and treated with sodium bisulfite to give the compounds of Formula IX which are isolated by standard procedures. Alternatively, the ω-amino acid may be reacted with a cyclic anhydride of Formula A (see Formula Chart) wherein m has the meaning defined in Formula I in an aprotic solvent or in aqueous media in the presence of a tertiary amine to yield the compounds of Formula IX.

The compounds of Formula IX wherein X is $-CON(R_{14})-$ are prepared by reacting an appropriate alkylene dicarboxylic acid with an appropriate aminoalkylsulfonate by procedures well known in the art.

The compounds of Formula IX wherein X is oxygen are prepared using t-butyl ester of a carboxylic acid of the formula

$$\text{t-Bu}-OCO(CH_2)_n\text{-halo}$$

wherein n is as defined in Formula I and halo is Cl, Br or I. This ester is prepared by reacting an appropriate ω-halo alkanoic acid of formula $HOOC(CH_2)_n$-halo with isobutylene gas in a dry aprotic solvent in the presence of catalytic amounts of sulfuric acid. The butyl ester is reacted with an ω-hydroxyalkyl sulfonic acid of formula $HO(CH_2)_mSO_3H$ wherein m is as defined in Formula I in a dry aprotic solvent in the presence of a strong base such as potassium t-butoxide to yield an ether. The ether is isolated by standard methods well known in the art and the carboxylic acid is deprotected by treatment with trifluoroacetic acid. The compounds of Formula IX are isolated by removing trifluoroacetic acid and solvent under reduced pressure.

The compounds of Formula IX wherein IX is sulfur are prepared by reaction of an ω-mercaptocarboxylic acid of the formula $HOOC(CH_2)_nSH$ and an ω-bromoalkyl sulfonic acid of formula $Br(CH_2)_mSO_3H$ or a sulfone of Formula B wherein n and m are as defined in Formula I in water containing three equivalents of inorganic base. A water miscible organic solvent, such as THF, may be added if required to solubilize the reactants. After several hours at 30—50°C the reaction is complete and the sulfide is isolated by extractive methods to give the compounds of Formula IX.

The compounds of Formula IX wherein X is sulfoxide are obtained by treating the corresponding Formula IX compound wherein X is sulfur with sodium periodate in water at 0 to 10°C for ~10—20 hours. The aqueous solution is diluted with at least two volumes of acetonitrile, $NaIO_3$ precipitate is filtered out, and the product is isolated by standard methods. The compounds of Formula VIII wherein X is sulfone are obtained by treating the corresponding sulfur compound with 30% hydrogen peroxide in 50% acetic acid for several hours at room temperature. The product is again isolated by standard procedures.

The compounds of Formulas X and XI are prepared by general procedures well known in the art. The bis-acids of Formula XII and the aminoalkylsulfonates of Formula XIV are known in the art or are prepared by means well known in the art. Also, the other starting materials described hereinabove including the ω-halosulfonates, the compounds of Formula B, the ω-haloalcohols, the ω-amino acids, the compounds of Formula A, the ω-haloalkanoic acid esters, and the ω-hydroxyalkylsulfonic acids are commercially available, or are known in the art or prepared by procedures generally known in the art.

Preparation of Compounds of Formula I wherein $R_{11}$ is $Y'-(CH_2)_p-X'-(CH_2)_q-NR_{12}R_{13}$

When Y' is oxy, i.e., —O—, equimolar amounts of an amine of the formula $O_2N(C_6H_4)-OCO)-(CH_2)_p-X'-(CH_2)_qNR_{12}R_{13}$ (Formula XXIV) wherein $(C_6H_4)$ is 1,4-phenylene and p, q, X', $R_{12}$ and $R_{13}$ have the meanings defined in Formula I, and a parent steroid of the formula StOH wherein St has the meaning defined in Formula VII are reacted in a dry aprotic solvent such as tetrahydrofuran (THF), dimethylformamide (DMF) or dimethylsulfoxide (DMSO), in the presence of an acylation catalyst such as dimethylaminopyridine (DMAP or N-methylimidazole. Although the reaction may be performed at room temperature it is convenient to gently warm the reaction mixture to about 50°—60°C with stirring until all the activated carbonate ester is consumed. The product is isolated by poring the reaction mixture into water with the pH adjusted to 2—4, washing with an organic solvent, e.g., ether or ethyl acetate, then quickly adjusting the pH to 7—8 and extracting with an organic solvent such as ethyl acetate. The product is isolated by removing the solvent and purified by recrystallization or chromatographic techniques.

When Y' is sulfur, i.e., —S—, equimolar quantities of an appropriate thiol amino of the formula $HS(CH_2)_p-X'-(CH_2)_q-NR_{12}R_{13}$ (Formula XVII) wherein p, q, X', $R_{12}$ and $R_{13}$ have the meanings defined in Formula I, and a chloroformate derivative of the parent steroid represented by the formula StOCOCl (Formula XVIII) wherein St has the meaning defined in Formula VII with an equivalent quantity of a tertiary amine, such as triethylamine, are reacted in a dry aprotic solvent such as, THF, DMF or DMSO. The reaction mixture may be warmed gently if desired. The product is isolated by extraction with an organic solvent such as ethyl acetate or hexane and purified by crystallization or chromatography.

When Y' is a bond the compounds are prepared by reacting equimolar amounts of an amino acid of the formula $HOOC(CH_2)_p-X'-(CH_2)_q-NR_{12}R_{13}$ (Formula XIX) wherein p, q, X', $R_{12}$ and $R_{13}$ have the meanings defined in Formula I with a 21-iodo or 21-O-mesyl derivative of the parent steroid which may be represented respectively by the formulas St-Iodo (Formula XX) and St-O-mesyl (Formula XXI) wherein St has the meaning defined in Formula VII and mesyl means $-S(O_2)-CH_3$. When the 21-iodo steroid derivative is employed the reaction proceeds at room temperature, whereas when the 21-O-mesyl steroid derivative is used the reaction is heated. Preferably both reactions are heated to about 60—70°C. The

reaction is carried out in a dry aprotic solvent such as DMF in the presence of a sterically hindered tertiary amine such as diisopropylethylamine. The product is isolated by extraction with an organic solvent, suitably ethyl acetate, and purified by recrystallization or chromatography.

When Y' is a bond and X is —CON($R_{14}$)— the compounds may also be prepared by reacting equimolar amounts of a 21-iodo steroid derivative of Formula XX and a bis-acid of the formula HOOC—$(CH_2)_p$—COOH (Formula XXIa) wherein p has the meaning defined in Formula I in a dry aprotic solvent such as THF or DMF in the presence of a sterically hindered amine such as diisopropylethylamine with optional heating to give an intermediate of the formula St—OOC—$(CH_2)_p$—COOH (Formula XXII) which is activated by cooling to about −20° to −10°C and reacting with isobutyl chloroformate in the presence of a tertiary amine, such as triethylamine for about 10—20 minutes during which time the reaction mixture is permitted to warm. To the activated derivative of Formula XXII is added an appropriate diamine of the formula $R_{14}NH$—$(CH_2)_qNR_{12}R_{13}$ (Formula XXV) wherein q, $R_{12}$, $R_{13}$, and $R_{14}$ have the meanings defined in Formula I. This latter reaction is complete within an hour, and the product is isolated by standard procedures, e.g., extraction with an appropriate organic solvent, such as ethyl acetate and purified by crystallization and/or chromatography.

Alternatively when Y is a bond and X is —CON($_{14}$)—, to the above obtained activated derivative of Formula XXII is added p-nitrophenol in the presence of a tertiary amine such as triethylamine to give a stable intermediate of the formula StOOC$(CH_2)_p$—COO$(C_6H_4)$—$NO_2$ (Formula XXIII) wherein St has the meaning defined in Formula VII, and $(C_6H_4)$ is 1,4-phenylene and p has the meaning defined in Formula I. The intermediate of Formula XXIII is then reacted with a molar equivalent of an amine of Formula XXV in a dipolar aprotic solvent such as THF or DMF in the presence of a base such as pyridine. The Formula I product is then isolated by extraction with an organic solvent, such as, ethyl acetate and purified by crystallization and/or chromatography.

The compounds of Formula XXIV wherein X' is —CON($R_{14}$)— are prepared by heating to about 60°C a suitable aliphatic lactone, such as, propiolactone, γ-butyrolactone, δ-valerolactone, ε-caprolactone, etc., as q in Formula XXV increases in length, with an equimolar amount of an aliphatic diamine of Formula XXV in an aprotic solvent such as DMSO, DMF or THF to give the acyclic amide which is isolated by diluting the reaction mixture with acidified water, washing with an immiscible solvent, such as ethyl acetate and adjusting the pH to about 12. The product is extracted with an organic solvent such as ethyl acetate, and the solvent is removed under reduced pressure to give the amide. The amide is reacted with p-nitrophenylchloroformate in a dry aprotic solvent such as THF in the presence of pyridine and isolated by standard procedures to give the compounds of Formula XXIV or used without isolation to form compounds of Formula I.

The compounds of Formula XXIV wherein X' is —N($R_{14}$)—CO— are prepared by reacting an appropriate N,N dialkyl amino alkanoic acid having an alkylene chain length of from 1 to 5 carbon atoms with a chloroformate ester, such as isobutyl chloroformate, in a dry chilled aprotic solvent, such as THF or DMF, in the presence of a tertiary amine to give the carboxylate-activated amino acid. This solution is then added dropwise with stirring to a second solution containing an equimolar amount of an amino alcohol of the formula HO—$(CH_2)_p$—NH($R_{14}$) wherein p and $R_{14}$ have the meanings defined in Formula I. An amide is obtained and any ester formed by reaction at the wrong end of the amino alcohol is eliminated by selective hydrolysis. The product is isolated by standard extractive methods, and the oily product is taken up in a dry aprotic solvent and treated with p-nitrophenylchloroformate in the presence of pyridine to give the compounds of Formula XXIV which may be isolated by standard procedures.

The compounds of Formula XXIV wherein X' is oxygen are prepared by reacting a suitable hydroxyalkoxyalkyl halide of the formula HO—$(CH_2)_p$—O$(CH_2)_q$— halide wherein p and q have the meanings defined in Formula I and halide is, e.g., chloride or bromide with an amine of the formula $HNR_{12}R_{13}$ wherein $R_{12}$ and $R_{13}$ are as defined in Formula I in a dry aprotic solvent with a catalytic amount of NaI present to yield an amino alcohol. After purifying the amino alcohol by extractive methods, it is taken up in a dry aprotic solvent and reacted with p-nitrophenylchloroformate in the presence of pyridine to give a reactive mixed p-nitrophenyl carbonate ester of Formula XXIV.

To prepare the compounds of Formula XXIV wherein X' is sulfur, an aliphatic ω-halo alcohol of the formula $(HO(CH_2)_p$ halo wherein p is as defined in Formula I and halo is chloro or bromo is reacted with an aliphatic thiol of the formula $(HS(CH_2)_qNR_{12}R_{13}$ wherein q, $R_{12}$ and $R_{13}$ are as defined in Formula I, to give a sulfide. The reaction is carried out in a partially aqueous solvent with a slight excess of NaOH and a reducing agent, e.g., sodium bisulfite, to inhibit disulfide formation. The product is isolated by extractive methods. This product may be oxidized at this stage to give a sulfoxide or sulfone if desired, or it may be maintained in the sulfide form. To form the sulfoxide, i.e., X' is —S(O)—, the sulfide amino alcohol is treated with one equivalent of sodium metaperiodate in aqueous lower alcohol at 0°C. When oxidation is complete the sodium iodate is filtered out and the sulfoxide isolated by standard procedures. To form the sulfone, i.e., X is —$S(O_2)$—, the sulfide amino alcohol is dissolved in a large excess of 90% formic acid and heated to about 70°C for several minutes. After cooling to room temperature the solution is treated with 30% hydrogen peroxide. Oxidation proceeds through the sulfoxide to the sulfone. When the oxidation is complete, most of the formic acid is removed under reduced pressure, and the remaining residue is taken up in methanolic HCl. After one hour the mixture is concentrated under reduced pressure to give the desired sulfone-linked amino alcohol as the HCl salt. Final purification is achieved by recrystallization or by

chromatography if needed. The sulfur-linked amino alcohol is then converted to a reactive mixed carbonate ester by combining it with an equimolar quantity of p-nitrophenylchloroformate in an aprotic solvent with added pyridine to give the compounds of Formula XXIV which may be isolated by standard procedures.

The compounds of Formula XXIV wherein X' is a bond are prepared by reacting an amino alkanol of the formula $HO(CH_2)_p—NR_{12}R_{13}$ wherein p, $R_{12}$ and $R_{13}$ are as defined in Formula I with p-nitrophenylchloroformate in a dry aprotic solvent, such as, THF in the presence of an amine, such as, triethylamine. The amino alkanol compounds are known in the art or are prepared by generally known procedures by treatment of an appropriate ω-iodoalkanol with an amine of the formula $NHR_{12}R_{13}$ wherein $R_{12}$ and $R_{13}$ are as defined in Formula I.

The compounds of Formula XVII wherein X' is a bond are prepared by reacting equimolar amounts of an ω-haloalkylamine of the formula halo-$(CH_2)_p—NR_{12}R_{13}$ wherein halo is halogen and p, $R_{12}$ and $R_{13}$ are as defined in Formula I and thiourea in propylene glycol at an elevated temperature. When the halide has been displaced, the isothiouronium salt is cleaved by adding an amine such as tetraethylene pentamine and continuing to apply heat. When the free thiol has formed, this product is isolated by extractive means or by distillation under reduced pressure.

The compounds of Formula XIX wherein X' is a bond are known in the art or are prepared by procedures well known in the art.

To prepare the compounds of Formula XVII wherein X' is $—CON—(R_{14})—$ an ω-haloalkyl$C_{2-9}$-carboxylic acid is reacted with equimolar quantities of triethylamine and isobutylchloroformate at −10°C in an aprotic solvent, preferably THF. The solution is allowed to warm to room temperature and a diamine of the formula $NH(R_{14})(CH_2)_qNR_{12}R_{13}$ wherein $R_{14}$, $R_{12}$, $R_{13}$ and q have the meanings defined in Formula I is added. After about 30 minutes the amide product is isolated by extractive procedures. This product is then reacted with an equimolar amount of thiourea in propylene glycol at an elevated temperature. When the halide has been displaced, the isothiouronium salt is cleaved by adding an amine such as tetraethylene pentamine and continuing to apply heat. When the free thiol has formed, this product is isolated by extractive means or by distillation under reduced pressure.

To prepare compounds of Formula XVII wherein X' is $—N(R_{14})CO—$ an amino acid of the formula $HOOC(CH_2)_qRR_{12}R_{13}$ wherein q, $R_{12}$ and $R_{13}$ are as defined in Formula I is activated by reaction with isobutylchloroformate in a chilled dry aprotic solvent, such as THF, with sufficient triethylamine to take up the liberated HCl. This solution is allowed to warm to room temperature and is then added dropwise under nitrogen to a solution containing an amino alcohol of the formula $HO(CH_2)_pNH—(R_{14})$ wherein p and $R_{14}$ are as defined in Formula I. The amide thus obtained is purified by standard procedures. This amide is then dissolved in pyridine and is treated with methane sulfonyl chloride to give the terminal mesyl group. The pyridine is removed under reduced pressure, and the product is heated with a 10% molar excess of thiourea in propylene glycol. When the displacement of the mesyl group by thiourea is complete the resulting isothiouronium salt is cleaved by heating with added tetraethylenepentamine to give the compounds of Formula XVII which are isolated by extractive procedures or by distillation.

The compounds of Formula XVII wherein X' is oxygen are prepared by reacting an N,N-disubstituted amino alcohol of the formula $HO(CH_2)_qNR_{12}R_{13}$ wherein q, $R_{12}$ and $R_{13}$ are as defined in Formula I with an equimolar quantity of sodium hydride in DMF to form the sodium alkoxide. This solution is then added dropwise to a large molar excess of an aliphatic $C_{2-9}$ dihalide or a dimesylate in DMF. If the halogen groups are chloride, sodium iodide is added as a catalyst. When ether formation is complete, the desired mono ether is isolated by extractive procedures then treated with thiourea in refluxing 95% ethanol to yield the isothiouronium salt. This salt is cleaved by treating the solution with a slight molar excess of sodium hydroxide solution and continuing to reflux the mixture under nitrogen. The amino thiol is then isolated from the reaction mixture by extractive procedures to give the compounds of Formula XVII.

The compounds of Formula XVII wherein X' is sulfur are prepared as follows. An N,N-disubstituted amino thiol of the formula $HS(CH_2)_qNR_{12}R_{13}$ wherein q, $R_{12}$ and $R_{13}$ are as defined in Formula I is dissolved in a lower alcohol and treated with a slight molar excess of NaOH. This solution is then added dropwise to a large molar excess of a dibromide of the formula $Br(CH_2)_qBr$ wherein p is an integer from 2 to 9, in an aprotic solvent such as DMF or THF. The desired mono-sulfide is isolated by standard extractive procedures. At this stage, the sulfide could be oxidized, if desired, to give either the sulfoxide or the sulfone. To prepare the compounds of Formula XVII wherein X' is sulfoxide the sulfide obtained above is treated with sodium metaperiodate in a lower aqueous alcohol by procedures analogous to those described hereinabove in connection with the preparation of compounds of Formula XXIV. To prepare the compounds of Formula XVII wherein X' is sulfone the sulfide is dissolved in glacial acetic acid and treated with 30% hydrogen peroxide thus oxidizing the sulfide through the sulfoxide to the sulfone. Whether or not further oxidation is elected, the subsequent steps are the same. The sulfur-linked amino bromide is treated with an equimolar amount of thiourea in refluxing 95% ethanol to yield an isothiouronium salt. This salt is cleaved by the addition of concentrated base to yield the free thiol. Upon acidification and extractive workup the compounds of Formula XVII are obtained.

The steroid chloroformates of Formula XVIII are prepared by reacting the parent 21 hydroxy steroid with a molar excess of phosgene in THF in a chilled reaction vessel which is then allowed to warm to room temperature. After about one hour the solution is concentrated under reduced pressure and the chloroformate precipitates out.

The compounds of Formula XIX wherein X' is —$N(R_{14})CO$— are prepared by reacting an aminoacid of the formula $HN(R_{14})(CH_2)_p$—COOH with a ω-bromoalkanoyl chloride wherein the alkanoyl moiety contains from 2 to 6 carbon atoms in an aqueous solvent at a pH of about 10 after which the pH is adjusted to about 3. The thus formed amide is extracted with an organic solvent such as ethyl acetate and isolated by procedures generally known in the art then taken up in an aprotic solvent such as THF or DMF and treated with an amine of the formula $HNR_{12}R_{13}$ wherein $R_{12}$ and $R_{13}$ have the meanings defined in Formula I to give the compounds of Formula XIX which are isolated by standard procedures.

The compounds of Formula XIX wherein X' is —$CON(R_{14})$— are prepared by reacting an appropriate alkylene dicarboxylic acid with an appropriate alkylenediamine by procedures well known in the art.

The compounds of Formula XIX wherein X' is oxy are prepared as follows. A t-butyl ester of a carboxylic acid of the formula t-bu-$OCO(CH_2)_{p-1}$—$CH_2R_b$ wherein p is as defined in Formula I and $R_b$ is a leaving group such as chloro, bromo, iodo, O-mesyl or O-tosyl is treated with an ω-hydroxy amine of the formula $HO(CH_2)_qNR_{12}R_{13}$ wherein q, $R_{12}$ and $R_{13}$ are as defined in Formula I, e.g., 2-diethylamino ethanol, and an equimolar amount of a strong non-nucleophilic base, e.g., potassium t-butoxide, in a dry aprotic solvent. e.g., THF, to yield the ether coupled promoiety. If the displaceable group is chloro or bromo, NaI may be added as a catalyst. When the ether formation is complete the product is isolated by extractive methods. The t-butyl ester is hydrolyzed by treatment with toluene sulfonic acid in an organic solvent, e.g., toluene, or with anhydrous trifluoroacetic acid to give the compounds of Formula XIX.

The compounds of Formula XIX wherein X' is sulfur are prepared by reaction of an ω-mercaptocarboxylic acid of the formula $HOOC(CH_2)_pSH$ and an ω-halo amine of the formula halo$(CH_2)_qNR_{12}R_{13}$ wherein p, q, $R_{12}$ and $R_{13}$ are as defined in Formula I and halo is chloro or bromo, in aqueous base containing a reducing agent, such as $K_2S_2O_5$. The pH is maintained at 10—12 by addition of base if necessary. A water miscible organic solvent, such as THF, may be added if required to solubilize the ω-halo-amine. When the reaction is complete the sulfide is isolated by extractive methods to give the compounds of Formula XIX.

The compounds of Formula XIX compound wherein X' is sulfur are obtained by treating the corresponding Formula XIX compound wherein X' is sulfur with sodium periodate in a lower aqueous alcohol as described hereinabove. The compounds of Formula XIX wherein X' is sulfone are obtained by treating the corresponding sulfur compound with hydrogen peroxide in 50% acetic acid by procedures analogous to those described hereinbefore.

The compounds of Formulas XX and XXI are prepared by general procedures well known in the art. The bis-acids for Formula XXIa and the alkylenediamines of Formula XXV are known in the art or are prepared by means well known in the art.

The ω-mercaptocarboxylic acids employed hereinbefore are obtained by treating an acid of the formula $HOOC(CH_2)_pR_c$ wherein $R_c$ is chloro, bromo, iodo, O-mesyl or O-tosyl and p is 2 to 9 with thiourea in a refluxing lower alcohol to give the isothiouronium salt which is subsequently cleaved by addition of aqueous base under reducing conditions to give the free thiol group.

The ω-haloamines employed hereinabove wherein m is other than 2 are obtained by adding a secondary amine of the formula $HNR_{12}R_{13}$ wherein $R_{12}$ and $R_{13}$ are as defined in Formula I portionwise to a molar excess of an appropriate 1,ω-alkylenedihalide. Generally the reaction mixture is heated and if the halide is chloride, an iodide salt may be added as a catalyst. The ω-haloamines wherein q is 2 are commercially available.

Preparation of Compounds of Formula I wherein $R_{11}$ is Z—$(CH_2)_r$Q

When Z is a bond, Q is $R_{15}$—$CH_2COOH$ and $R_{15}$ is —S—, —S(O)— or —$S(O)_2$— the compounds are prepared by reacting a steroid of the formula St—$X_5$ (Formula XXVI) wherein St has the meaning defined in Formula VII and $X_5$ is —$OSO_2CH_3$ or iodo, with a molar excess of a compound of the formula $HOOC(CH_2)_r$—$R_{15}$—$CH_2COOH$ (Formula XXVII) wherein $R'_{15}$ is —S—, —S(O)—, or —$S(O)_2$— and r is an integer from 2 to 9. The reaction is carried out in a polar aprotic solvent such as DMF or §MSO in the presence of at least 2 moles of an appropriate base per mole of the compound of Formula XXVII. The most preferred base is a bicyclic amidine such as 1,8-diazabicyclo[5.4.0]undec-7-ene (DBU).

Compounds of Formula XXVII wherein $R'_{15}$ is —$SO_2$— are prepared by treating a compound of the formula $HOCO(CH_2)_rSCH_2$—COOH (Formula XXVIII) with a strong oxidant such as 1:1 mixture of glacial acetic acid and 30% hydrogen peroxide. Compounds of Formula XXVII wherein $R_{15}$ is —SO— are prepared by treating a compound of Formula XXVIII with an equimolar amount of sodium periodate ($NaIO_4$) in aqueous methanol at 0 to 10°C for approximately one day. The reaction should be monitored to prevent over oxidation to the sulfone. Compounds of Formula XXVIII are prepared by reacting one equivalent of a compound of the formula $HOCO(CH_2)_rBr$ (Formula XXIX) with one equivalent of mercaptoacetic acid in water in the presence of three equivalents of strong base such as NaOH or KOH.

When Z is a bond, Q is $R_{15}$—$CH_2COOH$ and $R_{15}$ is —$N(R_{16})SO_2$— the compounds are prepared by treating a compound of the formula HOOC—$(CH_2)_rN(R_{16})SO_2CH_2COOH$ (Formula XXX) wherein r and $R_{16}$ are as defined in Formula I with one equivalent of a compound of Formula XXVI wherein $X_5$ is iodo, in a polar aprotic solvent such as dimethyl formamide, dimethyl sulfoxide or tetrahydrofuran in the presence of at least two equivalents of a bicyclic amidine such as 1,8-diazabicyclo[5.4.0]undec-7-ene or a sterically

hindered tertiary amine such as diisopropylethylamine. Preferably the reaction is carried out at room temperature using two equivalents of DBU.

Compounds of Formula XXX are prepared by treating compounds of the formula $R_aOOC(CH_2)_rN(R_{16})SO_2CH_2COOR_a$ (Formula XXXI) with aqueous mineral acid.

In Formula XXXI r and $R_{16}$ are as defined in Formula I and $R_a$ is a lower alkyl($C_1$—$C_4$) straight or branched chain. Compounds of Formula XXXI are prepared by treating an amino acid ester of the formula $R_aOOC(CH_2)_rN(R_{16})H$ where $R_a$, r and $R_{16}$ are as defined in Formula XXXI with a sulfonyl chloride of the formula $ClSO_2CH_2COOR_a$ wherein $R_a$ is as defined in Formula XXXi in a polar aprotic solvent in the presence of pyridine as a catalyst. The amino ester compounds are prepared by refluxing an amino acid of the formula $HOOC(CH_2)_rN(R_{16})H$ wherein r and $R_{16}$ are as defined in Formula I in an appropriate lower alcohol in the presence of a catalytic amount of sulfuric acid or anhydrous hydrochloric acid. The amino acids are known in the art or are obtained by treating an acid of the formula $HOOC(CH_2)_rL$ wherein L is Cl, Br, I, O-mesyl or O-tosyl with an amine of the formula $R_{16}NH_2$. The sulfonyl chloride compounds are prepared by treating a sulfoacetic acid of the formula $HSO_3CH_2COOR_a$ wherein $R_a$ is as defined above with thionyl chloride in an aprotic solvent or neat with excess thionyl chloride. Dimethylformamide may be added as a catalyst. The sulfoacetic acids are prepared by esterification of sulfoacetic acid in a refluxing lower alcohol.

When Z is a bond, Q is $R_{15}$—$CH_2COOH$ and $R_{15}$ is —$SO_2N(R_{16})$— the compounds are prepared by condensing a bis-acid of the formula $HOOC(CH_2)_rSO_2N(R_{16})CH_2COOH$ (Formula XXXII) wherein r and $R_{16}$ are as defined in Formula I with a compound of Formula XXVI wherein $X_5$ is iodo in a polar aprotic solvent in the presence of at least two equivalents of DBU or a hindered tertiary amine per equivalent of compound of Formula XXXII. The compounds of Formula XXXII are prepared by acid or base hydrolysis of the corresponding bis ester, i.e., a compound of formula $R_aOOC(CH_2)_rSO_2N(R_{16})CH_2COOR_a$ wherein $R_{16}$, r and $R_a$ are as defined hereinabove, and the bis ester is obtained by condensing an amine ester of the formula $H(R_{16})$ $NCH_2COOR_a$ with sulfonyl chloride of the formula $R_aOOC(CH_2)_rSO_2Cl$ in a polar aprotic solvent such as dimethyl formamide, tetrahydrofuran or dimethylsulfoxide in the presence of pyridine as a catalyst. The sulfonyl chloride is obtained by treating an acid of the formula $HOOC(CH_2)_rR_b$ wherein $R_b$ is, e.g., Cl, Br, I, O-mesyl or O-tosyl with sodum sulfite in aqueous methanol or ethanol at reflux to give the sulfonic acid $HOOC(CH_{2n}SO_3H$ which is further refluxed in an anhydrous lower alcohol to give the carboxy ester derivative which is treated with excess thionyl chloride in the presence of a catalytic amount of dimethyl formamide.

When Z is —O—, Q is $R_{15}CH_2COOH$ and $R_{15}$ is S, S(O) or $S(O)_2$, the compounds are prepared by reacting a steroid StOH wherein St is as defined in Formula VII with a compound of the formula $R_cOCOO(CH_2)_r$—$R'_{15}$—$CH_2COOR_g$ (Formula XXXIII) wherein $R_c$ is p-nitrophenyl, $R'_{15}$ is S, S(O) or $S(O)_2$, and r is an integer of from 2 to 9, and $R_g$ is $CH_3$ of 2,2,2-trichloroethyl in a polar aprotic solvent such as tetrahydrofuran, dimethylformamide or 4-dimethylsulfoxide in the presence of an acylation catalyst such as dimethylaminopyridine (DMAP) or N-methylimidazole and subsequently acid hydrolyzing the resulting ester to the corresponding acid. The Formula XXXIII compounds are prepared by treating an alcohol of the formula $HO(CH_2)_r$—$R'_{15}$—$CH_2COOR_g$ wherein $R'_{15}$, r, and $R_g$ are as defined above with equimolar amounts of p-nitrophenyl chlorocarbonate and a tertiary amine, e.g., triethylamine or pyridine in an aprotic solvent such as acetone, chloroform or tetrahydrofuran. The alcohols wherein $R'_{15}$ is —S— are obtained by reacting one equivalent of a compound of the formula $HO(CH_2)_rR_b$ wherein r is 4 to 9 and $R_b$ is Cl, Br, I, O-mesyl or O-tosyl with one equivalent of mercapto-acetic acid in water in the presence of sodium hydroxide or potassium hydroxide. The thus obtained compounds of the formula $HO(CH_2)_r$—S—$CH_2COOH$ are esterified, e.g., by treatment with a catalytic amount of a strong acid such as, sulfuric acid or toluenesulfonic acid in methanol at reflux or by treatment with 2,2,2-trichloroethanol in the presence of a catalytic amount of a mineral acid at 65 to 95°C. Following esterification the sulfur compounds can be oxidized to the sulfone by treatment with an equimolar amount of $NaIO_4$ in an aqueous alcohol at 0 to 10°C or to the sulfoxide by treatment with potassium hydrogen persulfate in aqueous alcohol. These oxidation steps may convert the carboxy methyl ester to the free acid and thus the resulting sulfone and sulfoxide can be reesterified as generally described above.

When Z is —O—, Q is —$R_{15}$—$CH_2COOH$, and $R_{15}$ is —$SO_2N(R_{16})$— the compounds are prepared by treating a compound of the formula $R_cOCOO$—$(CH_2)_rSO_2N(R_{16})CH_2COOCH_3$ (Formula XXXIV) wherein $R_{16}$ and r are as defined in Formula I and $R_c$ is p-nitrophenyl with a corticosteroid of the formula StOH wherein St is as defined in Formula VII in a polar aprotic solvent such as dimethylformamide, tetrahydrofuran, or dimethylsulfoxide in the presence of one equivalent of a tertiary amine such as pyridine or triethylamine and a catalytic amount of an acylation catalyst such as 4-dimethylaminopyridine or N-methylimidazole and selectively hydrolyzing the resulting ester to the acid by treating the ester with an aqueous solution of a strong acid such as hydrochloric or sulfuric. The Formula XXXIV compounds are prepared by treating a sulfonyl chloride of the formula $R_cOCOO(CH_2)_rSO_2Cl$ wherein r and $R_c$ are as defined above with two equivalents of the methyl ester or the 2,2,2-trichloroethyl ester of glycine or N-alkyl($C_1$—$C_4$) glycine in a suitable aprotic solvent such as tetrahydrofuran, dimethylformamide or dioxane. The sulfonyl chlorides are obtained by reacting an alcohol of the formula $HO(CH_2)_rR_b$ wherein r and $R_b$ are as defined hereinabove with a sulfite salt such as sodium sulfite in an aqueous lower alkanol at reflux to give compounds of the formula $HO(CH_2)_rSO_3Na$ which are reacted with p-nitrophenylchloroformate in a dry polar aprotic solvent

such as dimethylformamide or dimethylsulfoxide in the presence of a suitable amount of a tertiary amine such as trialkylamine or pyridine at 0 to 20°C to give compounds of the formula $R_cOCOO(CH_2)_r$—$R_d$ wherein $R_c$ is p-nitrophenyl, r is 4—9, and $R_d$ is a trialkyl($C_1$—$C_4$) ammonium or pyridinium which are treated with thionyl chloride either using excess thionyl chloride as solvent or using an aprotic solvent such as dimethylformamide.

When Z is —O—, Q is $R_{15}CH_2COOH$ and $R_{15}$ is —$N(R_{16})SO_2$— the compounds are prepared by treating a steroid of the formula StOH wherein St has the meaning defined in Formula VII with a compound of the formula $R_cOCOO(CH_2)_rN(R_{16})SO_2CH_2COOCH_3$ (Formula XXXV) wherein r and $R_{16}$ are as defined in Formula I and $R_c$ is p-nitrophenyl in a dry polar solvent such as dimethyl formamide or dimethylsulfoxide in the presence of an acylation catalyst such as DMAP or N-methylimidazole. The reaction will proceed at room temperature but is preferably carried out at about 40 to 50°C. The resulting ester is then selectively hydrolyzed with an aqueous acid such as hydrochloric, sulfuric or methanesulfonic. The Formula XXXV compounds are prepared by reacting p-nitrochloroformate with an alcohol ester of the formula $HO(CH_2)_rN$—$(R_{16})SO_2CH_2COOCH_3$ in a dry polar aprotic solvent in the presence of a tertiary amine. The alcohol esters are obtained by reacting a sulfonyl chloride of the formula $ClSO_2CH_2COOR_g$ wherein $R_g$ has the meaning defined hereinabove with an amino alcohol of the formula $HO(CH_2)_rNH$—$(R_{16})$ in an aprotic solvent and a stoichiometric amount of a tertiary amine. The amino alcohols are commercially available or prepared by reacting a primary amine with a halo alcohol, $HO(CH_2)$halo, and the sulfonyl chloride is prepared by well known procedures.

When Z is a bond and Q is CO—COOH the compounds are prepared by treating a steroid of the Formula XXVI wherein $X_5$ is iodo with a slight molar excess of a compound of the formula $HOCO(CH_2)_rCOCOOH$ (Formula XXXVI) in a polar aprotic solvent such as dimethylformamide or dimethylsulfoxide at room temperature in the presence of two molar equivalents of an organic base such as a tertiary amine but more preferably a bicyclic amidine such as DBU. The Formula XXXVI compounds are obtained by treating appropriate diesters of dicarboxylic acids with one equivalent of diethyloxalate in the presence of one equivalent of sodium ethoxide in ethanol, or preferably in an aprotic solvent such as diethylether to give after aqueous workup intermediate triesters of the formula

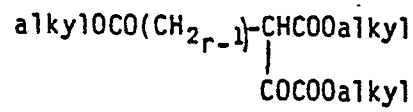

wherein alkyl has 1 to 4 carbon atoms, which are then treated with 4N HCl at 60—70°C for 5 to 10 hours to give the α-ketodicarboxylic acids.

The compounds of Formula I wherein Z is —O— and Q is —CO—COOH are prepared by treating a steroid of the formula StOH wherein St is as defined in Formula VII with a small molar excess of a compound of the formula $R_cOCOO(CH_2)_rCOCOOR_g$ (Formula XXXVII) wherein r is as defined in Formula I, $R_c$ is p-nitrophenyl, and $R_g$ has the meaning defined hereinabove in a polar aprotic solvent at 40 to 50° in the presence of one equivalent of organic base such as DMAP or a mixture of DMAP and pyridine and selectively hydrolyzing the resulting ester with aqueous acid. The compounds of Formula XXXVII are prepared by treating compounds of the formula $(HO(CH_2)_rCOCOOH$ (Formula XXXVIII) with two equivalents each of triethylamine and p-nitrophenylchlorocarbonate in a suitable solvent such as tetrahydrofuran at 0°C for 20 minutes then adding excess methanol of 2,2,2-trichloroethanol and one additional equivalent of triethylamine and allowing the mixture to warm to room temperature. The compounds of Formula XXXVIII are obtained by treating a lactone of the formula

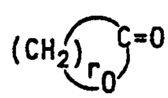

Formula XXXIX

with aqueous potassium hydroxide and treating the resulting potassium alkanoate salt with iodoacetamide to give compounds of the formula $HO(CH_2)_rCOOCH_2CONH_2$ which are treated with a stoichiometric amount of chlorotriphenylmethane in dry pyridine at 100°C for one hour to give compounds of the formula $R_eO(CH_2)_rCOOCH_2CONH_2$ wherein r is 2 to 8 and $R_e$ is triphenylmethyl. The triphenylmethyl derivatives are treated with aqueous base to give $R_eO(CH_2)_rCOOH$ which compounds are treated with excess thionyl chloride then heated at 150°C to 200°C for about two hours in the presence of excess cuprous cyanide to give $R_eO(CH_2)_rCOCN$ which compounds are treated with concentrated HCl for several days to give $HO(CH_2)_rCOCOOH$ compounds.

The compounds of Formula I wherein Z is a bond and Q is —$CON(R_{17})CH(R_{18})COOH$ are prepared by activating the carboxylic acid of a compound of the formula St—O—CO—$(CH_2)_rCOOH$ (Formula XLI) by treatment with stoichiometric amounts of isobutylchloroformate and triethylamine in a dry aprotic solvent at −10° to 0°C for 15 to 20 minutes, then adding an appropriate amino acid along with one equivalent of pyridine or triethylamine. Appropriate amino acids for this reaction and the one described below are glycine, sarcosine, alanine, aspartic acid, proline, glutamic acid, serine, threonine, cysteine, methionine, tyrosine, or glycylglycine. The compounds of Formula XLI are prepared by treating a compound of Formula XXVI, i.e., $StX_5$, with a stoichiometric amount of a sterically hindered tertiary amine such as diisopropylethylamine and a large excess of a dicarboxylic acid of the formula $HOOC(CH_2)_rCOOH$ in a polar aprotic solvent. When $X_5$ in Formula XXVI is iodo the reaction is carried out at room temperature and when $X_5$ is O-mesyl the reaction is carried out at about 45° to 60°C.

When Z is —O— and Q is $-CON(R_{17})CH(R_{18})COOH$ the compounds are prepared by treating a steroid StOH wherein St has the meaning defined in Formula VII with a compound of the formula $R_cOCOO(CH_2)_rCON(R_{17})CH(R_{18})COOCH_3$ (Formula XL) wherein r, $R_{17}$ and $R_{18}$ are as defined in Formula I and $R_c$ is p-nitrophenyl, in a polar aprotic solvent such as dimethylformamide or dimethylsulfoxide at 40° to 50°C in the presence of one equivalent of a tertiary amine such as pyridine and a catalytic amount of dimethylaminopyridine or N-methylimidazole and subsequently hydrolyzing the thus formed methyl ester derivative to the corresponding free acid using aqueous acid. The Formula XL compounds are prepared by treating a lactone of Formula XXXIX with a methyl ester or a 2,2,2-trichloroethyl ester of an appropriate amino acid as identified above in a polar aprotic solvent in the presence of one equivalent of a non-nucleophilic base at elevated temperature to give compounds of the formula $HO(CH_2)_rCON(R_{17})CH(R_{18})COOCH_3$ which are treated with a slight excess of p-nitrophenylchlorocarbonate in a dry aprotic solvent at 0 to 20°C in the presence of a stoichiometric amount of pyridine or a tertiary amine.

The methyl ester of the compounds described herein can be hydrolyzed to the free acid by heating in aqueous acid by well known procedures. The 2,2,2-trichloroethyl ester of the compounds described hereinabove can be converted to the free acid by treatment with zinc and acetic acid as generally described in J. Am. Chem. Soc. *88*, 852 (1966). The salts of the compounds of Formula I, are prepared by treating the acid with a suitable base as generally described hereinabove.

As indicated hereinbefore the various parent steroid starting materials, i.e., StOH and $StX_5$ are known in the art or are prepared by procedures well known in the art.

It may be desirable to protect various functions found on the 21-hydroxy steroid starting material, e.g. ketone groups or hydroxy groups at positions other than C—21. Protection and subsequent deprotection of functional groups on the parent steroid is accomplished by procedures well known in the art and/or as illustrated in the specific Examples which follow.

### Example 1

N-methyltaurine amide of 17α-hydroxy-4,9(11)-pregnadiene-3,20-dione-21-hemisuberate
[Formula I: $R_1 = CH_3$; $R_2$ and $R_3$ form a double bond; C—1 is saturated; $R_4$, $R_5$, $R_6$ and $R_9$ = H;
$R_{10}$ = α-OH; $R_{11} = -(CH_2)_6-CON(CH_3)-(CH_2)_2-SO_3H$]

A 20.0 g sample (.058 mol) of 17α,21-dihydroxy-4,9(11)-pregnadiene-3,20-dione was dissolved in 100 ml of pyridine and treated with 9.94 g (.087 mol) of methanesulfonyl chloride. After five hours, the mixture was partitioned between methylene choride and water. The organic phase was dried with sodium sulfate and concentrated. An insoluble material was filtered after the partition. The NMR spectra of the filtered solid and the methylene extract were the same and were consistent with the 21-mesylate. The combined mesylate fractions were refluxed in 600 ml of acetonitrile with 20 g of sodium iodide for 1.5 hours. The reaction was filtered through Celite® and concentrated to dryness. The residue was crystallised from hexane and acetone to yield a 17.8 g first crop of iodide. A 4.74 g (0.0105 mol) sample of the iodide was dissolved in 15 ml of DMF.

5.00 g (.0314 mol) of the N-methyltaurine amide of suberic acid (sodium salt) in 50 ml of methanol was reacted with 3.6 ml of 4.4 M sodium methoxide in methanol. This disodium salt was concentrated to dryness and added to the DMF solution of the iodide. The reaction mixture of the iodide and the disodium salt was stirred for 20 hours at 40°C. The mixture was then treated with ethyl acetate. The crystals which resulted were filtered and then partitioned between methylene chloride/isopropanol and a pH 4 sodium sulfate aqueous solution. The organic phase was dried over sodium sulfate and concentrated. The residue was chromatographed on silica gel with a methanol/methylene chloride gradient (2% to 15%) to yield 370 mg of final product.

### Example 2

N-methyltaurine amide of 6α-fluoro-17α-hydroxypregna-1,4,9(11)-triene-3,20-dione-21-hemisuberate, sodium salt

Pivaloyl chloride (8.76 g, .073 mol) was reacted in 350 ml of pyridine with 34.22 g (.109 mol) of 8-oxo-[(2-sulfoethyl)methylamino]octanoic acid, monosodium salt. A 13.15 g (.0363 g) sample of 6α-fluoro-17α,21-dihydroxy-pregna-1,4,9(11)-triene-3,20-dione was added after 2 hours. The mixture was stirred for 7 days. The mixture was concentrated and partitioned between n-butanol and aqueous sodium sulfate. The organic phase was dried over sodium sulfate and concentrated. The residue was chromatographed on silica gel (2% methanol/methylene chloride to 15%). The product was dissolved in methylene chloride and n-butanol and was washed with sodium sulfate in water. The organic phase was dried and concentrated. The residue was triturated with ether to yield 14.56 g of product which foamed at 130°C. HPLC analysis on C—18 showed 92% purity. Anal. Calcd. for $C_{33}H_{45}NFSNaO_9$. Calcd/Found: C, 58.83/58.11; H, 6.73/7.01; N, 2.08/2.17.

Example 3

N-methyltaurine amide of 6α-fluoro-16β-methyl-17α-hydroxypregna-1,4,9(11)-triene-3,20-dione-21-hemisuberate, sodium salt

A mixture of 41.06 g (.131 mol) of 8-oxo-[(2-sulfoethyl)methylamino]octanoic acid, monosodium salt, and 10.49 g (.087 mol) of pivaloyl chloride were stirred in 400 ml of pyridine for 2.5 h. A 15.78 g (.0436 mol) sample of 17α,21-dihydroxy-16β-methyl-6α-fluoropregna-1,4,9(11)-triene was added and the reaction was stirred for 20 hours. The mixture was concentrated and partitioned between n-butanol, water, sodium sulfate and sodium bicarbonate (.087 mol). The organic phase was dried over sodium sulfate and concentrated. The residue was chromatographed on silica gel (2% methanol/methylene chloride to 12%) to yield, after triturating to a white solid with ether, a 20.58 g pure cut whose mass spec was consistent with the product, mp to a glass 110–114°C. Anal. Calcd. for $C_{32}H_{43}NFSO_9Na.5H_2O$. Calcd/Found: C, 57.47/55.88; H, 6.63/6.74; N, 2.09/2.90; $H_2O$ 1.35/.97.

Example 4

N-methyltaurine amide of 6α-fluoro-1,4,9(11),16-tetraene-3,20-dione-21-hemisuberate, sodium salt

A 16.23 g (.0517 mol) sample of 8-oxo-[(2-sulfoethyl)methylamino]octanoic acid, monosodium salt, and 4.99 g (.0414 mol) of pivaloyl chloride in 120 ml of pyridine were stirred in an ice-bath for 3.5 hours. 21-Hydroxy-6α-fluoropregna-1,4,9(11),16-tetraene (6.70 g, .0207 mol) was added. The mixture was stirred for 19 hours and was then concentrated. The residue was poured into water which was saturated with sodium sulfate. Carbon dioxide was bubbled into the solution. The acidified mixture was partitioned between n-butanol and aqueous sodium sulfate. The organic phase was dried for 2h over magnesium sulfate. The product was chromatographed on silica gel (5% methanol/methylene chloride to 10% with 1% acetic acid) to yield, after ether trituration, 5.72 g of a tacky solid. This material was treated with 0.76 g of sodium bicarbonate in the usual way. The sodium salt was triturated with acetonitrile and ether to yield 4.24 g of product, mp 218—220°C. Anal. Calcd. for $C_{32}H_{41}NO_8FSNa.8H_2O$. Calcd/Found: C, 58.58/55.56; H, 6.54/6.10; N, 2.13/1.97; $H_2O$, 2.20/1.97.

FORMULA CHART

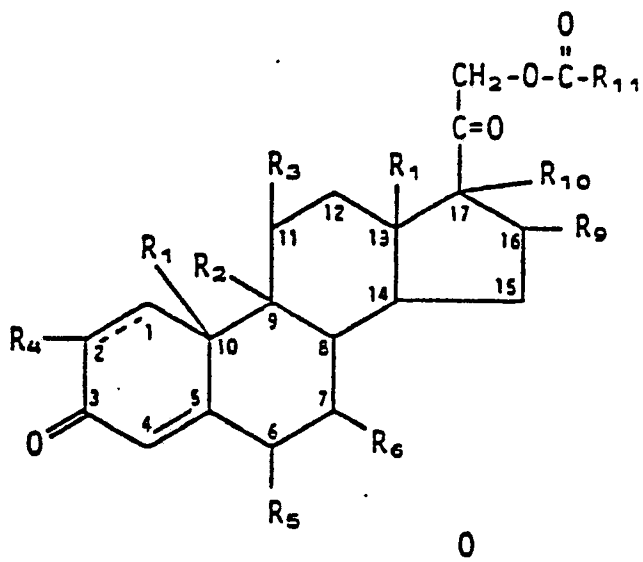

Formula I

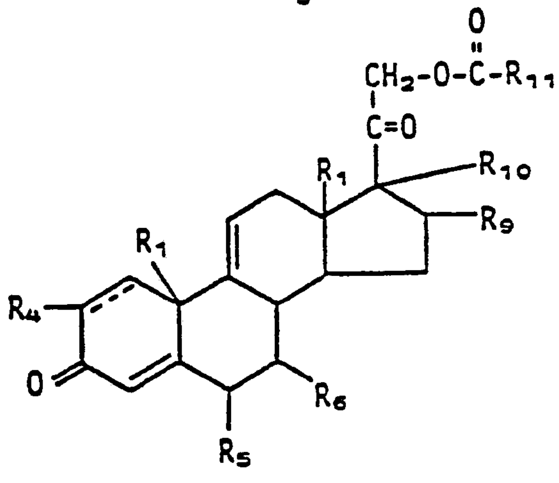

Formula II

FORMULA CHART (continued)

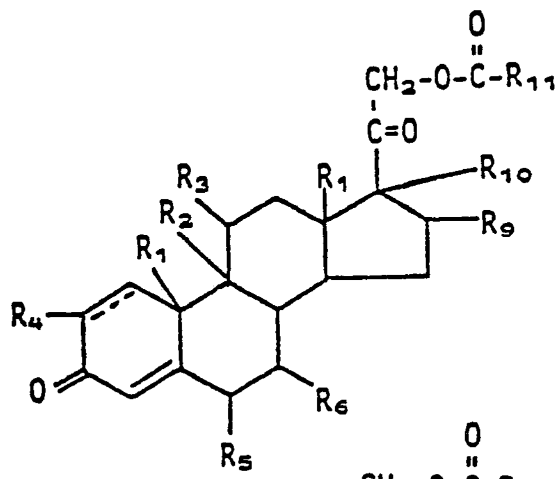

Formula III

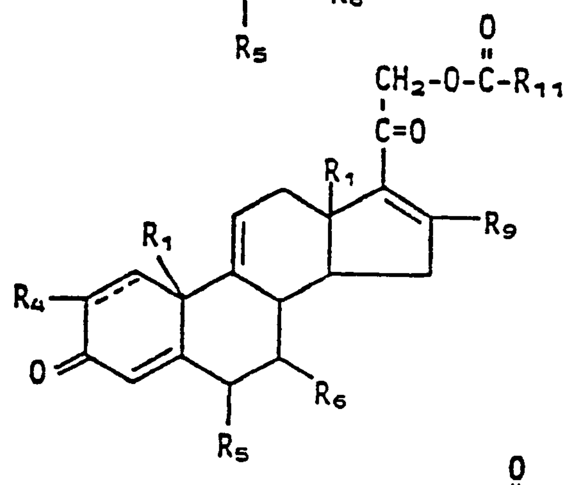

Formula IV

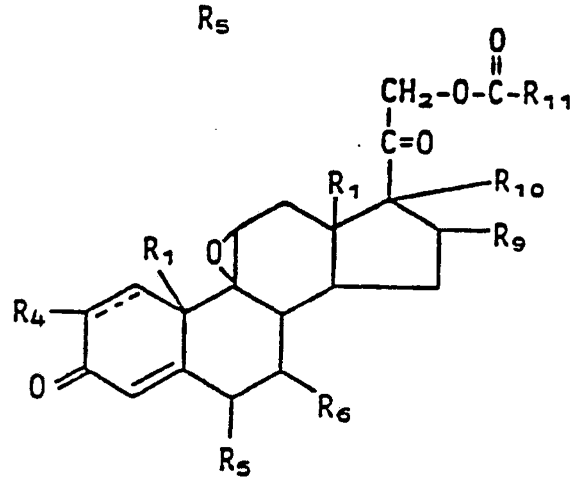

Formula V

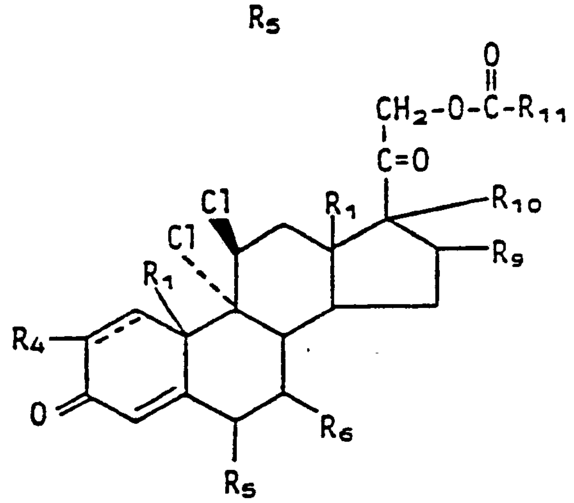

Formula VI

FORMULA CHART (continued)

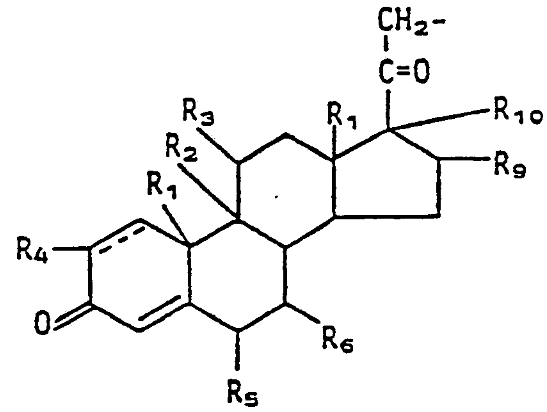

Formula VII

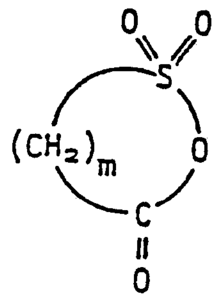

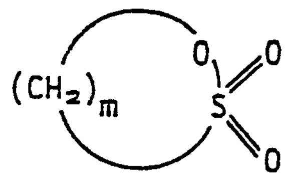

Formula A

Formula B

## Claims

1. A compound of the formula

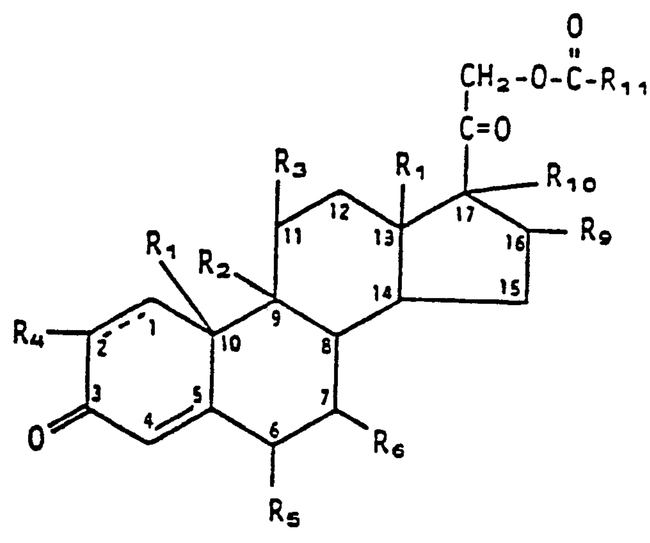

wherein --- indicates a single or double bond;

$R_1$ is $CH_3$ or $C_2H_5$;

either $R_2$ is H and $R_3$ is in the α-position and is OH, $C_{1-12}$ alkoxy, ($C_{1-4}$ alkyl)COO, aryl-COO, $(R)_2N$—COO or $R_7O$—COO, wherein the R's are independently selected from H and $C_{1-4}$ alkyl, $R_7$ is aryl or $C_{1-4}$ alkyl, and aryl is phenyl optionally substituted by 1 to 3 substituents selected from Cl, F, Br, $C_{1-3}$ alkyl, $C_{1-3}$ alkoxy, $C_{1-3}$ alkylthio, $Cl_3C$, $F_3C$, $NH_2$ and $NHCOCH_3$; or

$R_2$ is α-Cl and $R_3$ is β-Cl; or

$R_2$ and $R_3$ taken together are —O— bridging positions C—9 and C—11 or form a double bond between positions C—9 and C—11;

$R_4$ is H, $CH_3$, Cl or F;

$R_5$ is H, OH, F, Cl, Br or $CH_3$;

$R_6$ is H or $CH_3$;

$R_9$ is H, OH, $CH_3$, F or $=CH_2$;

either $R_{10}$ is H, OH or $CH_3$, or $R_{10}$ forms a second bond between positions C—16 and C—17; and

$R_{11}$ is $—Z(CH_2)_rQ$, $—Y—(CH_2)_n—X—(CH_2)_m—SO_3H$ or $—Y'—(CH_2)_p—X'—(CH_2)_q—NR_{12}R_{13}$, wherein Y is a bond or —O—; Y' is a bond, —O— or —S—; each of X and X' is a bond, $—CON(R_{14})—$, $—N(R_{14})—$, $—N(R_{14})CO—$, —O—, —S—, —S(O)—, or $—S(O_2)—$; $R_{14}$ is hydrogen or $C_{1-4}$ alkyl; either each of $R_{12}$ and $R_{13}$ is $(C_1—C_4)$ alkyl or $(C_1—C_4)$ monohydroxyalkyl or $NR_{12}R_{13}$ is pyrrolidino, piperidino, morpholino, thiomorpholino, piperazino or N—($C_{1-4}$ alkyl)piperazino; n is an integer of from 2 to 9; m is an integer of from 1 to 5; p is an integer of from 2 to 9; q is an integer of from 1 to 5; Z is a bond or —O—; r is an integer of from 2 to 9; and Q is

(1) $—R_{15}—CH_2COOH$ wherein $R_{15}$ is —S—, —S(O)—, $—S(O)_2—$, $—SO_2N(R_{16})—$ or $—N(R_{16})SO_2—$, and $R_{16}$ is hydrogen or $C_{1-4}$ alkyl;

(2) —CO—COOH;

(3) $—CONH—CHR_{18}—COOH$ wherein $R_{18}$ is H, $CH_3$, $—CH_2COOH$, $—CH_2CH_2COOH$, $—CH_2OH$, $—CH_2SH$, $—CH_2CH_2SCH_3$ or p-hydroxybenzyl;

(4) $—CONCH_3—CH_2—COOH$;

(5) (2-carboxy-1-pyrrolidinyl)carbonyl; or

(6) $—CONH—CH_2—CONH—CH_2COOH$;

with the provisos that:

(a) the total number of carbon atoms in $R_{16}$ and $(CH_2)_r$ is not greater than 10;

(b) when n is 2, $R_{14}$ is not H;

(c) the sum of m and n is not greater than 10:

(d) the sum of p and q is not greater than 10;

(e) when X is a bond, the sum of m and n is from 5 to 10;

(f) when X' is a bond, the sum of p and q is from 4 to 9;

(g) when $R_4$ is Cl or F, the C—1 position is saturated; and

(h) when $R_9$ is $=CH_2$, $R_{10}$ does not form a second bond between positions C—16 and C—17;

or a pharmaceutically-acceptable quaternary ammonium or other salt thereof.

2. A compound of claim 1, wherein $R_{10}$ forms a second bond between positions C—16 and C—17.

3. A compound of claim 1, wherein $R_4$ is α-F; $R_5$ is α-F, α-Cl or β-OH; $R_9$ is α-OH or α-$CH_3$; and $R_{10}$ is α-OH or α-$CH_3$.

4. A compound of claim 1, wherein $R_4$ is H; $R_9$ is H, α-OH or $CH_3$; and $R_{10}$ is α-H or α-OH.

5. A compound of claim 4, wherein $R_1$ is $CH_3$; $R_5$ is α-$CH_3$, F or H; and $R_{16}$ is H.

6. A compound of claim 4 or claim 5, wherein $R_2$ and $R_3$ taken together form a double bond between positions C—9 and C—11.

7. A compound of claim 4 or claim 5, wherein $R_2$ and $R_3$ taken together are oxygen (—O—) bridging positions C—9 and C—11.

8. A compound of claim 4 or claim 5, wherein $R_2$ is H.

9. A compound of claim 1, which is:

N-methyltaurine amide of 17α-hydroxypregna-4,9(11)-diene-3,20-dione-21-hemisuberate, sodium salt;

N-methyltaurine amide of 6α-fluoro-17α-hydroxypregna-1,4,9(11)-triene-3,20-dione-21-hemisuberate, sodium salt;

N-methyltaurine amide of 6α-fluoro-17α-hydroxy-16β-methylpregna-1,4,9(11)-triene-3,20-dione-21-hemisuberate, sodium salt; or

N-methyltaurine amide of 6α-fluoropregna-1,4,9(11),16-tetraene-3,20-dione-21-hemisuberate, sodium salt.

10. A pharmaceutical composition comprising a sterile aqueous solution of a compound of any preceding claim.

**Patentansprüche**

1. Eine Verbindung der Formel

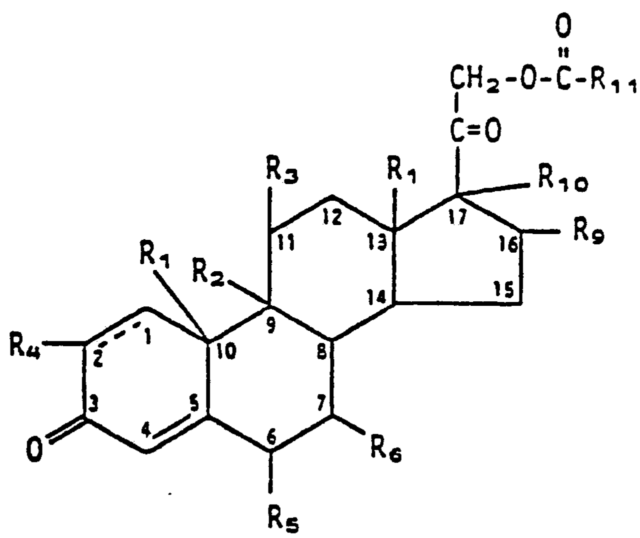

worin - - - eine Einfach- oder Doppelbindung anzeigt;

$R_1$ ist $CH_3$ oder $C_2H_5$;

entweder $R_2$ ist H und $R_3$ ist in α-Stellung und ist OH, $C_{1-12}$ Alkoxy, ($C_{1-4}$Alkyl)COO, Aryl-COO, $(R)_2N$—COO oder $R_7O$—COO, worin die R's unabhängig gewählt werden von H und $C_{1-4}$ Alkyl, $R_7$ ist Aryl oder $C_{1-4}$ Alkyl, und Aryl ist Phenyl wahlweise ersetzt durch 1 bis 3 Subtituenten, gewählt von Cl, F, Br, $C_{1-3}$ Alkyl, $C_{1-3}$ Alkoxy, $C_{1-3}$ Alkylthio, $Cl_3C$, $F_3C$, $NH_2$ und $NHCOCH_3$; oder

$R_2$ ist α-Cl und $R_3$ ist β-Cl; oder

$R_2$ und $R_3$ zusammengenommen bilden eine —O—Brücke zwischen Stellungen C—9 und C—11 oder eine Doppelbindung zwischen Stellungen C—9 und C—11;

$R_4$ ist H, $CH_3$, Cl oder F;

$R_5$ ist H, OH, F, Cl, Br oder $CH_3$;

$R_6$ ist H oder $CH_3$;

$R_9$ ist H, OH, $CH_3$, F oder $=CH_2$;

entweder $R_{10}$ ist H, OH oder $CH_3$, oder $R_{10}$ bildet eine zweite Bindung zwischen Stellungen C—16 und C—17; und

$R_{11}$ ist $—Z(CH_2)_rQ$, $—Y—(CH_2)_n—X—(CH_2)_m—SO_3H$ oder $—Y'—(CH_2)_p—X'—(CH_2)_q—NR_{12}R_{13}$, worin Y eine Bindung oder —O— ist; Y' ist eine Bindung, —O— oder —S—; jeder der X und X' ist eine Bindung, $—CON(R_{14})—$, $—N(R_{14})—$, $—N(R_{14})CO—$, —O—, —S—, —S(O)—, oder $—S(O_2)—$; $R_{14}$ ist Wasserstoff oder $C_{1-4}$ Alkyl; entweder jeder der $R_{12}$ und $R_{13}$ ist ($C_1$—$C_4$) Alkyl oder ($C_1$—$C_4$) Monohydroxyalkyl oder $NR_{12}R_{13}$ ist Pyrrolidino, Piperidino, Morpholino, Thiomorpholino, Piperazino oder N—($C_{1-4}$ Alkyl)piperazino; n ist eine ganze Zahl von 2 bis 9; m ist eine ganze Zahl von 1 bis 5; p ist eine ganze Zahl von 2 bis 9; q ist eine ganze Zahl von 1 bis 5; Z ist eine Bindung oder —O—; r ist eine ganze Zahl von 2 bis 9; und Q ist

(1) $—R_{15}—CH_2COOH$, worin $R_{15}$ is —S—, —S(O)—, $—S(O)_2—$, $—SO_2N(R_{16})—$ oder $—N(R_{16})SO_2—$, und $R_{16}$ Wasserstoff oder $C_{1-4}$ Alkyl ist;

(2) —CO—COOH;

(3) $—CONH—CHR_{18}—COOH$ worin $R_{18}$ is H, $CH_3$, $—CH_2COOH$, $—CH_2CH_2COOH$, $—CH_2OH$, $—CH_2SH$, $—CH_2CH_2SCH_3$ oder p-Hydroxybenzyl ist;

(4) $—CONCH_3—CH_2—COOH$;

(5) (2-Carboxy-1-pyrrolidinyl)carbonyl; oder

(6) $—CONH—CH_2—CONH—CH_2COOH$;

unter den Bedingungen, dass:

(a) die ganze Anzahl von Kohlenstoffatomen in $R_{16}$ und $(CH_2)_r$ nicht grösser als 10 ist;

(b) wenn n 2 ist, ist $R_{14}$ nicht H;

(c) die Summe von m und n nicht grösser als 10 ist;

(d) die Summe von p und q nicht grösser als 10 ist;

(e) wenn X eine Bindung ist, ist die Summe von m und n von 5 bis 10;

(f) wenn X' eine Bindung ist, ist die Summe von p und q von 4 bis 9;

(g) wenn $R_4$ Cl oder F ist, ist die C—1 Stellung gesättigt; und

(h) wenn $R_9$ $=CH_2$ ist, bildet $R_{10}$ nicht eine zweite Bindung zwischen Stellungen C—16 und C—17;

oder ein pharmazeutisch annehmbares quartäres Ammonium oder anderes Salz davon.

2. Eine Verbindung nach Anspruch 1, worin $R_{10}$ eine zweite Bindung zwischen Stellungen C—16 und C—17 bildet.

3. Eine Verbindung nach Anspruch 1, worin $R_4$ α-F ist; $R_5$ α-F, α-Cl oder β-OH ist; $R_9$ α-OH oder α-$CH_3$ ist; und $R_{10}$ α-OH oder α-$CH_3$ ist.

4. Eine Verbindung nach Anspruch 1, worin $R_4$ H ist; $R_9$ H, α-OH oder $CH_3$ ist; und $R_{10}$ α-H oder α-OH ist.

5. Eine Verbindung nach Anspruch 4, worin $R_1$ $CH_3$ ist; $R_5$ α-$CH_3$, F oder H ist; und $R_{16}$ H ist.

6. Eine Verbindung nach Anspruch 4 oder 5, worin $R_2$ und $R_3$ zusammengenommen eine Doppelbindung zwischen Stellungen C—9 und C—11 bilden.

7. Eine Verbindung nach Anspruch 4 oder 5, worin $R_2$ und $R_3$ zusammengenommen eine Sauerstoff(—O—)brücke zwischen Stellungen C—9 und C—11 bilden.

8. Eine Verbindung nach Anspruch 4 oder 5, worin $R_2$ H ist.

9. Eine Verbindung nach Anspruch 1, welche folgendes ist:

N-Methyltaurinamid des 17α-Hydroxypregna-4,9(11)-dien-3,20-dion-21-hemisuberats, Natriumsalz;

N-Methyltaurinamid des 6α-Fluor-17α-hydroxypregna-1,4,9(11)-trien-3,20-dion-21-hemisuberats, Natriumsalz;

N-Methyltaurinamid des 6α-Fluor-17α-hydroxy-16β-methylpregna-1,4,9(11)-trien-3,20-dion-21-hemisuberats, Natriumsalz; oder

N-Methyltaurinamid des 6α-Fluorpregna-1,4,9(11),16-tetraen-3,20-dion-21-hemisuberats, Natriumsalz.

10. Eine pharmazeutische Zusammensetzung, die eine sterile wässrige Lösung einer Verbindung einer der vorhergehenden Ansprüche enthält.

**Revendications**

1. Composé de formule

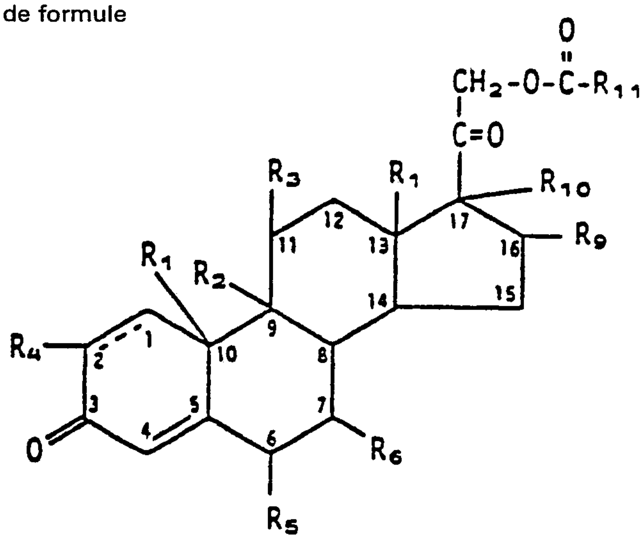

dans laquelle

--- désigne une liaison simple ou une double liaison;

$R_1$ représente un groupe $CH_3$ ou $C_2H_5$;

$R_2$ représente H et $R_3$ est en position α et représente un groupe OH, alkoxy en $C_1$ à $C_{12}$, alkyle en $C_1$ à $C_4$)COO, aryle-COO, $(R)_2N$—COO ou $R_7O$—COO, les groupes R' sont choisis, indépendamment, entre H et un groupe alkyle en $C_1$ à $C_4$, $R_7$ représente un groupe aryle ou alkyle en $C_1$ à $C_4$, et le groupe aryle est un groupe phényle facultativement substitué avec 1 à 3 substituants choisis entre Cl, F, Br, des groupes alkyle en $C_1$ à $C_3$, alkoxy en $C_1$ à $C_3$, alkylthio en $C_1$ à $C_3$, $Cl_3C$, $F_3C$, $NH_2$ et $NHCOCH_3$; ou bien

$R_2$ représente un groupe α-Cl et $R_3$ représente un groupe β-Cl, ou

$R_2$ et $R_3$, pris conjointement, représentent un atome —O— réalisant un pontage entre les positions C—9 et C—11 ou forment une double liaison entre les positions C—9 et C—11;

$R_4$ représente H, un groupe $CH_3$, Cl ou F;

$R_5$ représente H, un groupe OH, F, Cl, Br ou un groupe $CH_3$;

$R_6$ représente H ou un groupe $CH_3$;

$R_9$ représente H, un groupe OH, $CH_3$, F ou un groupe $=CH_2$;

$R_{10}$ représente H, un groupe OH ou $CH_3$, ou bien $R_{10}$ forme une seconde liaison entre les positions C—16 et C—17; et

$R_{11}$ représente un groupe $—Z(CH_2)_rQ$, $—Y—(CH_2)_n—X—(CH_2)_m—SO_3H$ ou $—Y'—(CH_2)_p—X'—(CH_2)_q—NR_{12}R_{13}$, dans lequel Y représente une liaison ou un groupe —O—; Y' représente une liaison, —O— ou —S—; chacun de X et X' représente une liaison, un groupe $—CON(R_{14})—$, $—N(R_{14})—$, $—N(R_{14})CO—$, —O—, —S—, —S(O)—, ou $—S(O_2)—$; $R_{14}$ représente l'hydrogène ou un groupe alkyle en $C_1$ à $C_4$; chacun de groupes $R_{12}$ et $R_{13}$ représente un groupe alkyle en $C_1$ à $C_4$ ou monohydroxy-alkyle en $C_1$ à $C_4$, ou bien $NR_{12}R_{13}$ représente un groupe pyrrolidino, pipéridino, morpholino,

thiomorpholino, pipérazino ou N-(alkyle en $C_1$ à $C_4$)pipérazino; *n* représente un nombre entier de 2 à 9; *m* représente un nombre entier de 1 à 5; *p* représente un nombre entier de 2 à 9; *q* représente un nombre entier de 1 à 5; Z représente une liaison ou —O—; *r* représente un nombre entier de 2 à 9; et Q représente

(1) un groupe $—R_{15}—CH_2COOH$ dans lequel $R_{15}$ représente —S—, —S(O)—, $—S(O)_2—$, $—SO_2N(R_{16})—$ ou $—N(R_{16})SO_2—$, et $R_{16}$ représente l'hydrogène ou un groupe alkyle en $C_1$ à $C_4$;

(2) un groupe —CO—COOH;

(3) un groupe $—CONH—CHR_{18}—COOH$ dans lequel $R_{18}$ représente H, un groupe $CH_3$, $—CH_2COOH$, $—CH_2CH_2COOH$, $—CH_2OH$, $—CH_2SH$, $—CH_2CH_2SCH_3$ ou p-hydroxybenzyle;

(4) un groupe $—CONCH_3—CH_2—COOH$;

(5) un groupe (2-carboxy-1-pyrrolidinyl)carbonyle; ou bien

(6) un groupe $—CONH—CH_2—CONH—CH_2COOH$;

sous réserve que:

(a) le nombre total d'atomes de carbone dans les groupes $R_{16}$ et $(CH_2)_r$ soit non supérieur à 10;

(b) lorsque *n* est égal à 2, $R_{14}$ ne représente pas H;

(c) la somme de *m* et *n* sopit non supérieure à 10;

(d) la somme de *p* et *q* soit non supérieure à 10;

(e) lorsque X représente une liaison, la somme de *m* et *n* soit comprise dans l'intervalle de 5 à 10;

(f) lorsque X' représente une liaison, la somme de *p* et *q* soit comprise dans l'intervalle de 4 à 9;

(g) lorsquef $R_4$ représente Cl ou F, la position C—1 soit saturée; et

(h) lorsque $R_9$ représente un groupe $=CH_2$, $R_{10}$ ne forme pas une seconde liaison entre les positions C—16 et C—17;

ou un de ses sels d'ammonium quaternaire ou autres sels pharmaceutiquement acceptables.

2. Composé suivant la revendication 1, dans lequel $R_{10}$ forme une seconde liaison entre les positions C—16 et C—17.

3. Composé suivant la revendication 1, dans lequel $R_4$ représente un groupe α-F; $R_5$ représente un groupe α-F, α-Cl ou β-OH; $R_9$ représente un groupe α-OH ou α-$CH_3$; et $R_{10}$ représente un groupe α-OH ou α-$CH_3$.

4. Composé suivant la revendication 1, dans lequel $R_4$ représente H; $R_9$ représente ou un groupe α-OH ou $CH_3$; et $R_{10}$ représente α-H ou un groupe α-OH.

5. Composé suivant la revendication 4, dans lequel $R_1$ représente un groupe $CH_3$; $R_5$ représente un groupe α-$CH_3$, F ou H; et $R_{16}$ représente H.

6. Composé suivant la revendication 4 ou la revendication 5, dans lequel $R_2$ et $R_3$, pris conjointement, forment une double liaison entre les positions $C_9$ et $C_{11}$.

7. Composé suivant la revendication 4 ou la revendication 5, dans lequel $R_2$ et $R_3$, pris conjointement, représentent un atome d'oxygène (—O—) réalisant un pontage entre les positions $C_9$ et $C_{11}$.

8. Composé suivant la revendication 4 ou la revendication 5, dans lequel $R_2$ représente H.

9. Composé suivant la revendication 1, qui est:

l'amide de N-méthyltaurine du sel de sodium du 21-hémisubérate de 17α-hydroxyprégna-4,9(11)-diène-3,20-dione;

l'amide de N-méthyltaurine du sel de sodium du 21-hémisubérate de 6α-fluoro-17α-hydroxyprégna-1,4,9(11)-triène-3,20-dione;

l'amide de N-méthyltaurine du sel de sodium du 21-hémisubérate de 6α-fluoro-17α-hydroxy-16β-méthylprégna-1,4,9(11)-triène-3,20-dione; ou

l'amide de N-méthyltaurine du sel de sodium du 21-hémisubérate de 6α-fluoroprégna-1,4,9(11),16-tetra-ène-3,20-dione.

10. Composition pharmaceutique comprenant une solution aqueuse stérile d'un composé suivant l'une quelconque des revendications précédentes.